(19) Europäisches Patentamt
European Patent Office
Office européen des brevets

(11) EP 0 973 038 B1

(12) **EUROPEAN PATENT SPECIFICATION**

(45) Date of publication and mention of the grant of the patent:
**23.08.2006 Bulletin 2006/34**

(51) Int Cl.:
***G01N 33/78*** *(2006.01)* ***G01N 33/566*** *(2006.01)*

(21) Application number: **99112316.7**

(22) Date of filing: **26.06.1999**

(54) **Method for measuring thyroglobulin**
Methode zur Messung von Thyroglobulin
Méthode de mesure de thyroglobuline

(84) Designated Contracting States:
**AT CH DE DK ES FI FR GB IT LI NL SE**

(30) Priority: **30.06.1998 JP 19979498**

(43) Date of publication of application:
**19.01.2000 Bulletin 2000/03**

(73) Proprietor: **Wako Pure Chemical Industries, Ltd.**
**Osaka 540-8605 (JP)**

(72) Inventors:
- **Kato, Ryoji**
**Matsumoto-shi,**
**Nagano 390-0804 (JP)**
- **Maruyama, Masayuki**
**Higashi chikuma-Gun,**
**Nagano 390-1301 (JP)**
- **Nakamura, Kenji**
**Amagasaki-Shi,**
**Hyougo 661-0963 (JP)**
- **Shimizu, Kayoko**
**Amagasaki-Shi,**
**Hyougo 661-0963 (JP)**
- **Satomura, Shinji**
**Osaka 540-8605 (JP)**

(74) Representative: **von Kreisler, Alek et al**
**Patentanwälte,**
**von Kreisler-Selting-Werner,**
**Bahnhofsvorplatz 1 (Deichmannhaus)**
**50667 Köln (DE)**

(56) References cited:
**WO-A-87/00289**

- **CHEMICAL ABSTRACTS, vol. 129, no. 22, 30 November 1998, Columbus, Ohio, US, abstract no. 287513k, MARUYAMA, M. ET AL.: "A method to differentiate between thyroglobulin derived from normal thyroid tissue and from thyroid carcinoma based on analysis of reactivity to lectins", page 254, columns 1-2; XP002900684 & Arch. Pathol. Lab. Med. 1998, 122(8), 715-720 (Eng),**
- **CHEMICAL ABSTRACTS, vol. 116, no. 13, 30 March 1992, Columbus, Ohio, USA, abstract no. 125879p, MIYAO, G. ET AL.: "Character- ization of thyroglobulin in human thyroid tumor tissue by lectin affinity electro- phoresis", page 595, column 1; XP002900685 & Iga ku no Ayumi 1992, 160(2), 161-2 (Japan),**
- **CHEMICAL ABSTRACTS, vol. 102, no. 11, 18 March 1985, Columbus, Ohio, USA, abstract no. 93837r, YAMAMOTO, K. ET AL.: "Phosphorylated high mannose- -type and hybrid-type oligo- saccharide chains of human thyroglobulin isolated from malignant thyroid tissue", page 417, column 2; XP002900686 & Biochem. Biophys. Acta 1985, 838(1), 84-91 (Eng),**
- **CHEMICAL ABSTRACTS, vol. 101, no. 15, 08 October 1984, Columbus, Ohio, USA, abstract no. 128176h, YAMAMOTO, K. ET AL.: "Structural changes of carbo- hydrate chains of human thyroglobulin accompanying malignant transformations of thyroid glands", page 507, column 2; XP002900687 & Eur. J. Biochem. 1984, 143(1), 133-44 (Eng),**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 973 038 B1

## Description

## BACKGROUND OF THE INVENTION

The present invention relates to a method for measuring thyroglobulin (hereinafter abbreviated as Tg), more particularly a method for measuring Tg(s) having a specific sugar chain structure, and further relates to a method for determining malignancy of thyroid carcinoma based upon an amount of Tg(s) having a specific sugar chain structure.

It has been known that Tg(s) is a sugar protein of molecular weight of 660,000 composed of a dimer of subunits having molecular weight of 330,000, which is secreted from a thyroid gland and that its content in cells or blood is increased in benign or malignant thyroid gland diseases. For this reason, the measurement of Tg(s) in blood has been used in progress observation after an operation on thyroid tumor. However, it is not possible to determine whether the thyroid tumor is benign or malignant on the basis of the Tg(s) content.

Thus, determination of malignancy of thyroid tumor has been conducted by sonography, punch aspiration biopsy, etc. However, there have been such problems that in the sonography, highly skilled diagnosis technique is required and in the punch aspiration biopsy, several days are required in the determination when culture the collected cells, and on the other hand highly skilled technique is required when the determination is conducted by observation of cells collected. Further, in the punch aspiration biopsy, there is also such a problem that differentiation of follicular adenoma from follicular carcinoma is difficult.

Further, it has been tried that benign or malignant of thyroid tumor is determined by specifying a sugar chain on the surface of the cell with the use of protein recognizing a sugar such as a lectin, and as the result, it has been found that there is observed a difference in sugar chain structures between benign and malignant thyroid carcinoma. In this method, however, each cell collected is reacted with a labeled protein recognizing a sugar chain and then an amount of the labeled material is confirmed under microscope, and thus this method is accompanied with such problems that skilled technique is required for the test and quantitative evaluation of the test result is difficult.

On the other hand, purified Tg(s) have been obtained from benign cell and malignant carcinoma cell and the structures of their sugar chains have been analyzed to find that the structures of sugar chains in the cells are different between the benign cell and the malignant carcinoma cell. However, also in this method, there is such a problem that a long time is required for the purification of Tg(s) from the cells collected and for the analysis of the structures of the sugar chains.

## BRIEF SUMMARY OF THE INVENTION

Under the situation as mentioned above, the problem to be solved by the present invention is to provide a method for easily and simply measuring various kinds of Tg(s) in samples originated from a living body, to provide a method for determining malignancy of thyroid tumor on the basis of the measured result and to provide a reagent for this purpose.

The present invention has been accomplished by the method, use and object as defined in the appended claims.

## BRIEF DESCRIPTION OF THE DRAWINGS

Fig.1 shows a ratio (%) of Tg(s) not bound to Concanavalin A related to the total Tg(s) in various kinds of thyroid tissue extracts obtained in Example 1.

Fig.2 shows a ratio (%) of Tg(s) not bound to *Ricinus communis* agglutinin-120 related to the total Tg(s) in various kinds of thyroid tissue extracts obtained in Example 2.

Fig.3 shows a ratio (%) of Tg(s) not bound to Concanavalin A related to the total Tg(s) in various kinds of thyroid tissue extracts obtained in Example 3.

Fig.4 shows a ratio (%) of Tg(s) not bound to *Lens culinaris* agglutinin related to the total Tg(s) in various kinds of thyroid tissue extracts upon using "anti Tg-86" as an anti Tg antibody, which was obtained in Example 4.

Fig.5 shows a ratio (%) of Tg(s) not bound to *Lens culinaris* agglutinin related to the total Tg(s) lectin in various kinds of thyroid tissue extracts upon using "anti Tg-78" as an anti Tg antibody, which was obtained in Example 4.

Fig.6 shows a ratio (%) of Tg(s) not bound to Concanavalin A related to the total Tg(s)in various kinds of thyroid tissue extracts

## DETAILED DESCRIPTION OF THE INVENTION

The present inventors have extensively studied to solve the above mentioned problems to reach a finding that

a total amount of Tg(s) and an amount of Tg(s) having a specific sugar chain structure or that of Tg(s) having a sugar chain structure other than the specific sugar chain structure in a living sample can be measured by using each one or more kinds of "proteins capable of specifically binding to Tg(s)" and "proteins capable of specifically binding to a specific sugar chain structure of Tg(s) having the specific sugar chain structure" , and have continued to study on the basis of this finding to reach an additional finding that the amount of Tg(s) having a specific sugar chain structure and/or that of Tg(s) having a sugar chain structure other than the specific sugar chain structure, or a ratio of Tg(s) having a specific sugar chain structure or that of Tg(s) having a sugar chain structure other than the specific sugar chain structure to total amount of Tg(s) in a living sample can advantageously be used for determining malignant of thyroid tumor, and on the basis of those findings, the present invention has been accomplished.

The "proteins capable of binding to a constant region of Tg(s)" of the present invention includes anti-Tg antibody capable of binding to a constant region of Tg(s), a receptor capable of binding to Tg(s), etc. The protein may be used alone or in a suitable combination of two or more thereof. The constant region of Tg(s) means a region having a structure which is common to all Tg(s) in a living sample. The "Tg-binding protein" of the present invention may have a capability of binding also to a region other than the constant region.

The"Tg-binding protein" includes one having a characteristic of "proteins capable of binding to Tg(s) but not capable of binding to Tg(s) to which the "proteins capable of specifically binding to a specific sugar chain structure of Tg(s) having the specific sugar chain structure" (hereinafter abbreviated as "a protein binding to a specific sugar chain") is already bound (hereinafter abbreviated as "a competitive Tg-binding protein"), one having a characteristic of "a protein capable of binding to all Tg(s) regardless whether a protein binding to a specific sugar chain structure is already bound thereto or not" (hereinafter abbreviated as "a non-competitive Tg-binding protein").

The anti-Tg antibody capable of binding to a constant region of Tg(s) may be any polyclonal antibodies prepared by immunizing an animal such as horse, sheep, rabbit, goat, rat and mouse with the substance to be measured according to a conventional method described in Tadashi Matsuhashi et al. "Meneki Jikkengaku Nyumon" 2nd. ed., Gakkai-Shuppan Center Ltd., 1981; or any monoclonal antibodies produced by hybridomas obtained by fusing cells from a tumor cell line of mouse with mouse spleen cells previously immunized with the substance to be measured according to a conventional cell fusion method established by G. Köhler and C. Milstein [Nature, 256, 495 (1975)].

The "protein binding to a specific sugar chain structure" of the present invention includes an antibody, a lectin, etc. which are capable of specifically binding to a specific sugar chain structure of Tg(s). More definitely, including an antibody reactive with Lewis type sugar chain such as anti-$Le^a$ antibody, anti-$Le^b$ antibody, anti-$Le^x$ antibody, anti-$Le^y$ antibody, anti-S-$Le^a$ antibody and other antibodies, a lectin capable of binding to L-fucose such as *Lotus tetragonolobus* agglutinin, a lectin capable of binding to D-galactose or N-acetyl-D-galactosamine such as *Arachis hypogoea* agglutinin, soybean agglutinin, *Ricinus communis* agglutinin and phytohemagglutinin, a lectin capable of binding to D-mannose such as Concanavalin A, *Lens culinaris* agglutinin and *Pisum sativum* agglutinin, a lectin capable of binding to N-acetylglucosamine such as wheat germ agglutinin and *Datura stramonium* agglutinin, a lectin capable of binding to sialic acid such as *Limulus polyphemus* agglutinin, etc., among which those capable of binding to D-galactose or N-acetyl-D-galagtosamie and those capable of binding to D-mannose are preferable. The lectin may be used alone or in a suitable combination of two or more thereof.

"The capable of binding to a sugar chain structure" in the above classification of the lectin means that a lectin once bound to an affinity column immobilized a suitable sugar chain can be eluted by this sugar. For instance, a lectin capable of binding to D-galactose or N-acetyl-D-galactosamine means that a lectin once bound to an affinity column can be eluted by this D-galactose or N-acetyl-D-galactosamine.

The antibody capable of specifically binding to a specific sugar chain structure of Tg(s) having the specific sugar chain structure may also be polyclonal antibodies or monoclonal antibodies prepared by a conventional manner as mentioned above.

The specific sugar chain structure in the present invention includes particularly (1) a sugar chain structure to which the above mentioned lectin can be bound and (2) a sugar chain structure contained in Tg(s) which is produced by carcinoma cell such as thyroid carcinoma. More particularly, the sugar chain structures described in documents such as Yamamoto, K., Eur. J. Biochem., vol. 143, 133-144, 1984 and so on can be mentioned.

The measurement method of the present invention is characterized in that various kinds of Tg(s) in a sample derived from a living body such as plasma, serum, cerebrospinal fluid, extracted solution of various kinds of living tissues and urine are measured by using "a Tg-binding protein" and "a protein binding to a specific sugar chain structure" in a suitable combination, and the definite object to be measured includes a total Tg(s) amount(s), an amount of Tg(s) having a specific sugar chain structure, an amount of Tg(s) having a sugar chain structure other than the specific sugar chain structure. Tg(s) is decomposed in a living body to give various kinds of fragments, and these fragments to which "a Tg-binding protein" and/or "a protein binding to a specific sugar chain structure" can be bound, among them, can also be the object to be measured in the present invention.

Those objects to be measured can be measured separately or simultaneously in one shot step.

The follows are working modes of the present invention.

(1) A method for measuring Tg(s), which comprises

① reacting a sample originated from a living body with either one of (a) a protein-(1) capable of binding to a constant region of Tg(s) or (b) a protein-(2) capable of specifically binding to a specific sugar chain structure of Tg(s) having the specific sugar chain structure to form a conjugate of [the protein-(1)]-[Tg] or [the protein-(2)]-[Tg],
② reacting the resulting conjugate with the other one of (a) the protein-(1) or (b) the protein-(2), which is labeled by a labeling substance, to form a conjugate of [the protein-(1)]-[Tg]-[the labeled protein-(2)] or [the protein-(2)]-[Tg]-[the labeled protein-(1)],
③ separating the conjugate from the free labeled protein (2) or the free labeled protein-(1), and
④ measuring an amount of the labeling substance in the conjugate formed in the step②, whereby an amount of a Tg(s) having the specific sugar chain structure is obtained.

(2) A method for measuring Tg(s), which comprises

① reacting a sample originated from a living body with a labeled protein-(2) capable of specifically binding.to a specific sugar chain structure of Tg(s) having the specific sugar chain structure and a protein-(3) capable of binding to all Tg(s) regardless whether the labeled protein-(2) is already bound thereto or not to form a conjugate of [the labeled protein-(2)]-[Tg]-[the protein-(3)], and
② separating the conjugate from the free labeled protein-(2), and
③ measuring an amount of the labeling substance in the conjugate formed in the step①, whereby an amount of a Tg(s) having the specific sugar chain structure is obtained.

(3) A method for measuring Tg(s), which comprises

① reacting a sample originated from a living body with a protein-(2) capable of specifically binding to a specific sugar chain structure of Tg(s) having the specific sugar chain structure to form a conjugate of [Tg]-[the protein-(2)],
② separating the conjugate from Tg(s) to which the protein-(2) is not bound, and
③ measuring an amount of the Tg(s) having the specific sugar chain structure in the conjugate.

(4) A method for measuring Tg(s), which comprises

① reacting a sample originated from a living body with a protein-(1) capable of binding to a constant region of Tg(s) to form a conjugate of [the protein-(1)]-[Tg],
② reacting the conjugate with both of a protein-(2) capable of specifically binding to a specific sugar chain structure of Tg(s) having the specific sugar chain structure, and then reacted with a labeled protein-(4) capable of binding to Tg(s) but not capable of binding to Tg(s) to which the protein-(2) is already bound, to form conjugate (a) of [the protein-(1)]-[Tg]-[the protein-(2)] and a conjugate (b) [the protein-(1)]-[Tg]-[the labeled protein-(4)],
③ separating the conjugates from the free labeled protein-(4), and
④ measuring an amount of the labeling substance in the conjugate (b) formed in the step②, whereby an amount of Tg(s) having a sugar chain structure other than the specific sugar chain structure is obtained.

(5) A method for measuring Tg(s), which comprises

① reacting a sample originated from a living body with a protein-(2) capable of specifically binding to a specific sugar chain structure of Tg(s) having the specific sugar chain structure, and then reacted with a protein-(4) capable of binding to Tg(s) but not capable of binding to Tg(s) to which the protein-(2) is already bound to form a conjugate of (a)[the protein-(2)]-[Tg] and a conjugate of (b)[the protein-(4)]-[Tg],
② separating the conjugate (b) from others,
③ reacting the conjugate (b) with a labeled protein-(1) capable of binding to a constant region of Tg(s) to form a conjugate (c) of [the protein-(4)]-[Tg]-[the labeled protein(1)],
④ separating the conjugate (c) from free labeled protein-(1), and
⑤ measuring an amount of the labeling substance in the conjugate (c) formed in the step③, whereby an amount of Tg(s) having a sugar chain structure other than the specific sugar chain structure.

(6) A method for measuring Tg(s), which comprises

① reacting a sample originated from a living body with both a protein-(2) capable of specifically binding to a specific sugar chain structure of Tg(s) having the specific sugar chain structure and a labeled protein-(4) capable

of binding to Tg(s) but not capable of specifically binding to a Tg(s) to which the protein-(2) is already bound to form a conjugate of (a)[the protein-(2)]-[Tg] and a conjugate of (b)[the labeled protein-(4)]-[Tg], and
② separating the conjugate (b) from the free labeled protein-(4), and
③ measuring an amount of the labeling substance in the conjugate(b) formed in the step③, whereby an amount of Tg(s) having a sugar chain structure other than the specific sugar chain structure is obtained.

(7) A method for measuring Tg(s), which comprises

① reacting sample originated from a living body with both a labeled protein-(1) capable of binding to a constant region of Tg(a) and a protein-(2) capable of specifically binding to a specific sugar chain structure of Tg(s) having the specific sugar chain structure to form conjugates of (a) [the labeled protein-(1)]-[Tg] and (b) [the labeled protein-(1)]-[Tg]-[the protein-(2)], and
② separating the each conjugates from the free labeled protein-(1), and
③ measuring the amounts of the labeling substances in each of the conjugate (a) and conjugate (b) formed in the step① are measured respectively, whereby amounts of Tg(s) having the specific sugar chain structure, Tg (s) having a sugar chain structure other than the specific sugar chain structure and total Tg(s) are obtained.

(8) A method for measuring Tg(s), which comprises

① reacting a sample originated from a living body with a protein-(2) capable of specifically binding to a specific sugar chain structure of Tg(s) having the specific sugar chain structure and a labeled protein-(3) capable of binding to all Tg(s) regardless whether the protein-(2) is already bound thereto or not, and a protein-(4) capable of binding to Tg(s) but not capable of binding to Tg(s) to which the protein-(2) is already bound to form conjugates of (a) [the labeled protein-(3)]-[Tg]-[the protein-(4)] and (b) [the labeled protein-(3)]-[Tg]-[the protein-(2)],
② removing the free labeled protein-(3), and separating the conjugates(a) and (b), and
③ measuring the amounts of the labeling substances in each conjugate(a) and (b) formed in the step①, whereby amounts of Tg(s) having the specific sugar chain structure, Tg(s) having a sugar chain structure other than the specific sugar chain structure and total Tg(s) are obtained.

(9) A method for measuring Tg(s), which comprises

① reacting a sample originated from a living body with a labeled protein-(1) capable of binding to a constant region of Tg(s) and a protein-(2) capable of specifically binding to a specific sugar chain structure of Tg (s) having the specific sugar chain structure and a protein-(4) capable of binding to Tg(s) but not capable of binding to Tg (s) to which the protein-(2) is already bound to form conjugates of (a) [the labeled protein-(1)]-[Tg]-[the protein-(4)] and [the labeled protein-(1)]-[Tg]-[the protein-(2)]
② removing the free labeled protein-(1), and separating the conjugate(a) and (b), and
③ measuring the amounts of the labeling substances in each conjugate (a) and (b)formed in the step①, whereby amounts of Tg(s) having the specific sugar chain structure, Tg(s) having a sugar chain structure other than the specific sugar chain structure and total Tg(s) are obtained.

The following are specific examples of the measurement methods.

I.Methods for measuring the objects separately

The objects to be measured, namely, a total Tg(s) amount, an amount of Tg(s) having a specific sugar chain structure and an amount of Tg(s) having a sugar chain structure other than the specific sugar chain structure are respectively measured by the following.

I-1. Measurement of a total Tg(s) amount

It can be measured by a per se known measurement method using "a Tg-binding protein".

I-2. Measurement of an amount of Tg(s) having a specific sugar chain structure

I-2-1) A method using "a Tg-binding protein" immobilized on an insoluble carrier

A "Tg-binding protein" immobilized on an insoluble carrier is reacted with a sample originated from a living body

such as plasma, serum, cerebrospinal fluid, extracted solution of various kinds of living tissues and urine, whereby the following immobilized conjugate is formed.

INSOLUBLE CARRIER – "a Tg-binding protein" –Tg

Then, unnecessary co-existing substances are removed by, for instance, washing, and the immobilized conjugate is reacted further with a "protein binding to a specific sugar chain structure" bound to a labeling substance (hereinafter abbreviated as "a labeled protein binding to a specific sugar chain structure"), whereby the following immobilized conjugate is formed.

INSOLUBLE CARRIER – "a Tg-binding protein" –Tg– "a labeled protein binding to a specific sugar chain structure"

Then, the immobilized conjugate product is, washed, for instance, to remove a free "labeled protein binding to a specific sugar chain structure" , and an amount of the labeling substance in the immobilized conjugate is measured after a suitable measurement method, and the resulting measured value is applied to a calibration curve showing a relationship between an amount of a labeling substance (measured value) and a concentration of Tg(s) which is previously obtained by measuring a standard solution containing a known amount of Tg(s) having a specific sugar chain structure after a similar method, whereby an amount of Tg(s) having a specific sugar chain structure in the sample can be obtained.

I-2-2) A method using "a protein binding to a specific sugar chain structure" immobilized on an insoluble carrier

A sample originated from a living body such as plasma, serum, cerebrospinal fluid, extracted solution of various kinds of living tissues and urine is reacted with "a protein binding to a specific sugar chain structure" immobilized on an insoluble carrier, whereby the following conjugate is formed.

INSOLUBLE CARRIER – "a protein binding to a specific sugar chain structure" –Tg

Then, unnecessary co-existing substances are removed, for instance, by washing, and further "a Tg-binding protein" bound to a labeling substance (hereinafter abbreviated as "a labeled Tg-binding protein") is reacted with the conjugate to give the following immobilized conjugate.

INSOLUBLE CARRIER – "a protein binding to a specific sugar chain structure" –Tg– "a labeled Tg-binding protein"

Then, the immobilized conjugate product is washed, for instance, to remove a free "labeled Tg-binding protein" and an amount of the labeling substance in the immobilized conjugate is measured after a suitable measurement method, and the resulting measured value is applied to a calibration curve showing a relationship between an amount of a labeling substance (measured value) and a concentration of Tg(s) which is previously obtained by measuring a standard solution containing a known amount of Tg(s) having a specific sugar chain structure after a similar method, whereby an amount of Tg(s) having a specific sugar chain structure in the sample can be obtained.

I-2-3) A method using "a labeled protein binding to a specific sugar chain structure" and high-performance liquid chromatography (HPLC), etc.

A sample originated from a living body such as plasma, serum, cerebrospinal fluid, extracted solution of various

kinds of living tissues and urine is reacted with "a labeled protein binding to a specific sugar chain structure" and "a non-competitive Tg-binding protein" to form the following conjugate in the sample.

"a labeled protein binding to a specific sugar chain structure" -Tg - "a non-competitive Tg-binding protein"

Then, the conjugate and a free "labeled protein binding to a specific sugar chain structure" are separated from each other by HPLC packed with a suitable packing agent, electrophoresis, etc. and an amount of the labeled substance in the conjugate is measured after a suitable method, and the resulting measured value is applied to a calibration curve showing a relationship between an amount of a labeling substance (measured value) and a concentration of Tg(s) which is previously obtained by measuring a standard solution containing a known amount of Tg(s) having a specific sugar chain structure after a similar method, whereby an amount of Tg(s) having a specific-sugar chain structure in the sample can be obtained.

I-3. Measurement of Tg(s) having a sugar chain structure other than the specific sugar chain structure

I-3-1) A method using a free "protein binding to a specific sugar chain structure"

At first, a sample originated from a living body such as plasma, serum, cerebrospinal fluid, extracted solution of various kinds of living tissues and urine is reacted with "a protein binding to a specific sugar chain structure" to form a conjugate of Tg(s) having a specific sugar chain structure and "a protein binding to a specific sugar chain structure" (hereinafter sometimes abbreviated as a " sugar chain-binding Tg"). Then, a sugar chain-binding Tg and Tg(s) to which a protein binding to a specific sugar chain structure is not bound, in other words, Tg(s) having a sugar chain structure other than the specific sugar chain structure (hereinafter abbreviated as a "non-binding Tg"), are removed from the sample by a per se known separation method such as centrifugation method, gel filtration method, molecular fraction membrane method and electrophoresis method, etc., whereby a sample containing only a non-binding Tg is prepared.

An amount of Tg(s) in thus obtained sample containing only a non-binding Tg is measured by a per se known method using "a Tg-binding protein" to give an amount of a non-binding Tg.

The sample containing only a non-binding Tg may also be prepared by treating the sample with affinity chromatography using "a protein binding to a specific sugar chain structure" immobilized on an insoluble carrier.

I-3-2) A method using "a competitive Tg-binding protein"

At first, "a Tg-binding protein" immobilized on an insoluble carrier is reacted with a sample originated from a living body such as plasma, serum, cerebrospinal fluid, extracted solution of various kinds of living tissues and urine to form the following immobilized conjugate.

INSOLUBLE CARRIER – "a Tg-binding protein" – Tg

Then, unnecessary co-existing substances are removed, for example, by washing, and the immobilized conjugate is reacted with "a protein binding to a specific sugar chain structure" and further with "a competitive Tg-binding protein" to which a labeling substance is bound (hereinafter abbreviated as"a labeled competitive Tg-binding protein") to give the following immobilized conjugate.

INSOLUBLE CARRIER – "a Tg-binding protein" – Tg – "a protein binding to a specific sugar chain structure"

INSOLUBLE CARRIER – "a Tg-binding protein" – Tg – "a labeled competitive Tg-binding protein"

Then, a free "labeled competitive Tg-binding protein" is removed by, for example, washing the immobilized conjugates, and an amount of the labeled substance in the immobilized conjugates is measured after a suitable method, and the resulting measured value is applied to a calibration curve showing a relationship between an amount of a labeling substance (measured value) and a concentration of Tg(s) which is previously obtained by measuring a standard solution containing a known amount of Tg(s) having a sugar chain structure other than the specific sugar chain structure, namely a non-binding Tg after a similar method, whereby an amount of a non-binding Tg in the sample can be obtained.

I-3-3) A method using"a competitive Tg-binding protein" immobilized on an insoluble carrier

At first, a sample originated from a living body such as plasma, serum, cerebrospinal fluid, extracted solution of various kinds of living tissues and urine is reacted with "a protein binding to a specific sugar chain structure" to form "a sugar chain-binding Tg" in the sample, and then the sample is reacted with "a competitive Tg-binding protein" immobilized on an insoluble carrier to form the following immobilized conjugate.

**INSOLUBLE CARRIER – “a competitive Tg-binding protein” – a non-binding Tg**

Then, unnecessary co-existing substances are removed, for instance, by washing, and the immobilized conjugate is reacted with "a labeled Tg-binding protein" to form the following immobilized conjugate.

**INSOLUBLE CARRIER – “a competitive Tg-binding protein” – a non-binding protein – “a labeled Tg-binding protein”**

Then, a free "labeled Tg-binding protein" is removed by, for instance, washing the immobilized conjugate, and an amount of the labeling substance in the immobilized conjugate is measured after a suitable method, and the resulting measured value is applied to a calibration curve showing a relationship between an amount of a labeling substance (measured value) and a concentration of Tg(s) which is previously obtained by measuring a standard solution containing a known amount of a non-binding Tg after a similar method, whereby an amount of a non-binding Tg in the sample can be obtained.

I-3-4) A method using "a labeled competitive Tg-binding protein" and HPLC, etc.

At first, a sample originated from a living body such as plasma, serum, cerebrospinal fluid, extracted solution of various kinds of living tissues and urine is reacted with "a protein binding to a specific sugar chain structure" to form a sugar chain-binding Tg. Then, the resulting sample is reacted with "a labeled competitive Tg-binding protein" to form the following conjugate.

a non-binding Tg - "a labeled competitive Tg-binding protein" Then, the conjugate is separated from a free "labeled

competitive Tg-binding protein" by HPLC packed with a suitable packing agent, electrophoresis, etc., and an amount of the labeling substance in the conjugate is measured by a suitable method, and the resulting measured value is applied to a calibration curve showing a relationship between an amount of a labeling substance (measured value) and a concentration of Tg(s) which is previously obtained by measuring a standard solution containing a known amount of a non-binding Tg after a similar method, whereby an amount of a non-binding Tg in the sample can be obtained.

Needless to say, an amount of Tg(s) having a specific sugar chain structure (sugar chain-binding Tg) can be obtained by subtracting an amount of Tg(s) having a sugar chain structure other than the specific sugar chain structure (a non-binding Tg) from a total Tg(s) amount, and an amount of Tg(s) having a sugar chain structure other than the specific sugar chain structure (a non-binding Tg) can be obtained by subtracting an amount of Tg(s) having a specific sugar chain structure (a sugar chain-binding Tg) from a total Tg(s) amount.

II. A method for measuring the objects simultaneously in one shot step

II-1. A method using "a labeled Tg-binding protein"and"a protein binding to a specific sugar chain structure"

This method can be conducted as follows after a method disclosed in Japanese Patent Publication-Kokai-No. 191027/1995.

Namely, at first, ① a sample originated from a living body such as plasma, serum, cerebrospinal fluid, extracted solution of various kinds of living tissues and urine is reacted with "a labeled Tg-binding protein" and "a protein binding to a specific sugar chain structure", or ② the sample is reacted with "a labeled Tg-binding protein" and then the resulting reaction solution is further reacted with "a protein binding to a specific sugar chain structure" added to the solution, whereby the following conjugates are formed.

"a labeled Tg-binding protein" -Tg

"a labeled Tg-binding protein" -Tg- "a protein binding to a specific sugar chain structure"

Then, the conjugates and a free "labeled Tg-binding protein" are separated from each other by using HPLC packed with a suitable packing agent, electrophoresis, etc. and amounts of the labeled substances in the respective conjugates are measured after a suitable method, and the resulting measured values are applied to a calibration curve showing a relationship between an amount of a labeling substance (measured value) and a concentration of Tg(s) which is previously obtained by measuring standard solutions containing a known amount of Tg(s) having a specific sugar chain structure and/or Tg(s) having a sugar chain structure other than the specific sugar chain structure after a similar method, whereby an amount of Tg(s) having a specific sugar chain structure, an amount of Tg(s) having a sugar chain structure other than a specific sugar chain structure and a total amount thereof, namely a total Tg(s) amount, in the sample can be obtained simultaneously in one step.

As the method for separation of conjugates and a free "labeled Tg-binding protein" from one another, HPLC is preferable because it is easily handled and can be conducted repeatedly.

As the "Tg-binding protein", a non-competitive one is preferable.

II-2. A method using "a non-competitive Tg-binding protein", "a competitive Tg-binding protein" and "a protein binding to a specific sugar chain structure"

At first, a sample originated from a living body such as plasma, serum, cerebrospinal fluid, extracted solution of various kinds of living tissues and urine is reacted with "a non-competitive Tg-binding protein" to which a suitable labeling substance is bound (hereinafter abbreviated as "a labeled non-competitive Tg-binding protein"), "a competitive Tg-binding protein" and "a protein binding to a specific sugar chain structure" to form the following conjugates.

"a labeled non-competitive Tg-binding protein" -Tg- "a competitive Tg-binding protein"

"a labeled non-competitive Tg-binding protein" -Tg- "a protein binding to a specific sugar chain structure"

Then, the conjugates and a free "labeled non-competitive Tg-binding protein" are separated from each other by using HPLC packed with a suitable packing agent, electrophoresis, etc. and amounts of the labeled substances in the respective conjugates are measured after a suitable method, and the resulting measured values are applied to a calibration curve showing a relationship between an amount of a labeling substance (measured value) and a concentration of various kinds of Tg(s) which is previously obtained by measuring standard solutions containing a known amount of Tg(s) having a specific sugar chain structure and/or Tg(s) having a sugar chain structure other than the specific sugar chain structure after a similar method, whereby an amount of Tg(s) having a specific sugar chain structure, an amount of Tg(s) having a sugar chain structure other than the specific sugar chain structure and a total amount thereof, namely a total Tg(s) amount, in the sample can be obtained simultaneously in one step. Further, by allowing "a non-competitive Tg-binding protein" having a different epitope from "the labeled non-competitive Tg-binding protein" to take a reaction at the same time, difference between the properties of the conjugates and those of serum ingredients can be increased, as a result of which influences by serum ingredients can be minimized to increase measurement accuracy. In this point of view, this technique is desirable.

As the method for separation of conjugates and a free "labeled non-competitive Tg-binding protein" from one another, HPLC is preferable because it can be conducted repeatedly.

II-3. A method using "a labeled Tg-binding protein" and "a competitive Tg-binding protein"

At first, ① a sample originated from a living body such as plasma, serum, cerebrospinal fluid, extracted solution of various kinds of living tissues and urine is reacted with "a labeled Tg-binding protein" , and further "a protein binding to a specific sugar chain structure" and "a competitive Tg-binding protein" are reacted with the above reaction solution to form the following conjugates.

"a labeled Tg-binding protein" -Tg- "a competitive Tg-binding protein"

"a labeled Tg-binding protein" -Tg- "a protein binding to a specific sugar chain structure"

Then, the conjugates and a free "labeled non-competitive Tg-binding protein" are separated from each other by using HPLC packed with a suitable packing agent, electrophoresis, etc. and amounts of the labeled substances in the respective conjugates are measured after a suitable method, and the resulting measured values are applied to a calibration curve showing a relationship between the measured values of a labeling substance and a concentration of various kinds of Tg(s) which is previously obtained by measuring standard solutions containing a known amount of Tg (s) having a specific sugar chain structure and/or Tg(s) having a sugar chain structure other than the specific sugar chain structure after a similar method, whereby an amount of Tg(s) having a specific sugar chain structure, an amount of Tg (s) having a sugar chain structure other than the specific sugar chain structure and a total amount thereof, namely a total Tg(s) amount, in the sample can be obtained simultaneously in one shot step. Further, by allowing "a Tg-binding protein" having a different epitope from "the labeled Tg-binding protein" to take a reaction after the reaction with "a labeled Tg-binding protein", difference between the properties of the conjugates and those of serum ingredients can be increased, as a result of which influences by serum ingredients can be minimized to increase measurement accuracy. In this point of view, this technique is desirable.

As the method for separation of conjugates and a free "labeled Tg-binding protein" from one another, HPLC is preferable because it is easily handled and can be conducted repeatedly.

The per se known measurement method using "a Tg-binding protein" can be conducted with the use of an anti Tg antibody, for example, as the "Tg-binding protein" after an immunological assay such as enzyme immunoassay (EIA), radioimmunoassay (RIA), enzyme-linked immunosorbent assay (ELISA), fluoroimmunoassay (FIA) and a method using HPLC (Japanese Patent Publication-Kokai-No. 301995/1997), and the measurement mechanism may be any of a sandwich method. a competitive method, a double antibody technique method, etc.

The insoluble carrier used for immobilizing various kinds of "a Tg-binding protein" and "a protein binding to a specific sugar chain structure" may be any of those so far used in the field of the above immunological assay and includes beads, tube, a special tray or microtiter plate in which many tubes are molded in a body, etc., which are made from metal, glass, ceramic, silicone rubber, synthetic polymer such as polystyrene, polyvinyl chloride, polypropylene, acryl resin and polymethylmethacrylate, etc., and an immobilizing method may be any of so far used in the field of immunological assay, such as physical adsorption methods and chemical binding methods.

The labeling substance to be bound to "the Tg-binding protein" and "the protein binding to a specific sugar chain structure" is bound includes alkali phosphatase, β-galactosidase, peroxidase, microperoxidase, glucoseoxidase, glucose-6-phosphate dehydrogenase, acetylcholinesterase, malic dehydrogenase, luciferase and other enzymes which are used in EIA, $^{99m}Tc$, $^{131}I$, $^{125}I$, $^{14}C$, $^{3}H$ and other radioisotopes which are used in RIA, fluoresceine, dansyl, fluorescamine, coumarin, naphthylamine and their derivatives and other fluorescent substances which are used in FIA, luciferin, isoluminol, luminol, bis (2,4,6-trifluorophenyl) oxalate and other luminescent substances, phenol, naphthol, anthracene and their derivatives and other substances which can absorb an ultarviolet light, 4-amino-2,2,6,6-tetramethylpiperidine-1-oxyl, 3-amino-2,2,5,5-tetramethylpyrrolidine-1-oxyl, 2,6-di-t-butyl- α-(3,5-di-t-butyl-4-oxo-2,5-cyclohexadien-1-ylidene)-p-tolyl oxyl and other oxyl group-containing compounds which have characteristics as spin-labeling agent.

The method for binding (labeling) the above labeling substance to "the Tg-binding protein" and "the protein binding to a specific sugar chain structure" is bound may be conducted after a per se known one so far conducted generally in per se known EIA, RIA, FIA, etc. As the labeling method, a conventional one using a reaction of avidin (or streptoavidin) with biotin may be used.

HPLC apparatus used in the measurement method using HPLC in the present invention may be any one so far used in this kind of field.

In the measurement method using HPLC of the present invention, for the purpose of clearer separation of a conjugate from a free "labeled Tg-binding protein" (or "labeled protein binding to a specific sugar chain structure"), use may be made of "the Tg-binding protein" or "the protein binding to a specific sugar chain structure" is bound, to which a substance enhancing the separation (hereinafter abbreviated as "separation enhancing substance") disclosed in Japanese Patent Publication-Kokai-No. 191027/1995, No.301995/1997, etc. is may be used.

The "separation enhancing substance" used for this purpose includes preferably proteins such as α-chymotripsinogen, β-galactosidase, lysozyme, cytochrome c and trypsin inhibitor; peptides containing an amino acid such as phenylalanin, proline, arginine, ricin, aspartic acid and glutamic acid; halogen atoms such as bromine, chlorine and iodine; synthetic polymers such as polyethylene glycol; polyamino acids such as polyglutamic acid, polyaspartic acid, polylysin, polyarginine, polyphenylalanin and polytyrosine; alkyl chains having 3 to 10 carbon atoms; fatty acids such as palmitic acid, oleic acid and stearic acid; chemical substances having a group reactive with "the Tg-binding protein" and "the protein binding to a specific sugar chain structure" is bound and showing hydrophobic property, such as N-(ε-maleimidocaproyloxy) succinimide (EMCS), N-succinimidyl-6-maleimidohexanoate, Bismaleimidohexane (BMH) and octylamine, peptides containing a strong acid residue such as 4-(p-maleimidophenyl) butylyl Ala-$(Tyr(SO_3H))_5$ and 4-(p-maleimidophenyl) butylyl Ala-$(Tyr(SO_3H))_8$ which are disclosed in Japanese Patent Publication-Kokai-No. 301995/1997, etc. The "separation enhancing substance" to be used can be selected considering characteristics (such as pH stability, hydrophobicity, solubility in water and isoelectric point) of the objects to be measured such as Tg(s), Tg-binding proteins and proteins to which a specific sugar chain is bound.

A method for binding"the separation enhancing substance"with "the Tg-binding proteins"and/or"the proteins binding to a specific sugar chain structure" can be conducted after (1) a per se known manner for binding a labeled substance with an antibody generally used in per se known EIA, RIA or FIA (e.g. Yuichi Yamamura "Ikagaku Jikken Koza Vol.8" 1st ed., NAKAYAMA-SHOTEN Ltd., 1971, Akira Kawano "Zusetsu Keikokotai"1st ed., Soft Science, Inc., 1983; and Eiji Ishikawa, Tadashi Kawai and Kiyoshi Miyai "Koso Men-eki Sokuteiho" 2nd. ed., IGAKU-SHOIN Ltd., 1982), (2) a per se known manner for modification and binding of a substance (e.g. Ikuzo Uritani; Kensuke Shimura, Michinori Nakamura and Masaru Funazu "Tanpakushitu-no Kagakushiushoku 〈Jo〉, 〈Ge〉" 1st ed., GAKKAO-SHUPPAN CENTER Ltd., 1981; Yuji Inada et al. "Poly (ethylene glycol) Shushoku Tanpakushitsu" Seikagaku vol. 62, No.11, pp. 1351-1362, Japanese Biochemical Association, 1990; and George H K. and Mark M. M. "DNA PROBES" STOCKTON PRESS, 1989), etc.

By a suitable combination of values of a total Tg(s) amount, an amount of Tg(s) having a specific sugar chain structure and Tg(s) having a sugar chain structure other than the specific sugar chain structure, which are obtained by the measurement method of the present invention, malignancy of thyroid tumor can be determined.

Namely, for instance, by obtaining a content of Tg(s) having a specific sugar chain structure or Tg(s) having a sugar chain structure other than the specific sugar chain structure in the total Tg(s), malignancy of thyroid tumor, in other words, as to whether the tumor is benign or malignant thyroid carcinoma, can be determined.

Further in detail, by obtaining a content of Tg(s) having a specific sugar chain structure or Tg(s) having a sugar chain structure other than the specific sugar chain structure in the total Tg(s) with the use of lectins capable of binding to D-galactose or N-acetyl-D-galactosamine such as *Arachis hypogoea* agglutinin, soybean agglutinin, *Ricinus communis* agglutinin and phytohemagglutinin, lectins capable of binding to D-mannose such as concanavalin A, *Lens culinaris* agglutinin and *Pisum sativum* agglutinin, etc., as the "protein binding to a specific sugar chain structure" , differentiation of benign thyroid adenoma or Graves' disease, etc. from papillary carcinoma etc., and differentiation of follicular adenoma from follicular carcinoma, etc. becomes possible on the basis of the result thus obtained.

For instance, diagnosis using the extract from a tissue sample can be performed, taking the following phenomenon into consideration;

(1) the ratio(%) of Tg(s) not bound to Con A related to the amount of total Tg(s) in sample from the papillary carcinoma is significantly higher than those in the sample from benign disease such as benign thyroid adenoma, Grave's disease, follicular adenoma or thyroid adenomatous.
(2) the ratio(%) of Tg(s) not bound to Con A, related to the amount of total Tg(s) in sample from the follicular carcinoma is significantly higher than those in the sample from follicular adenoma and normal.
(3) the ratio(%) of Tg(s) not bound to RCA120 related to the amount of total Tg(s) in sample from the papillary carcinoma is significantly higher than those in the sample from Grave's disease and normal.
(4) the ratio(%) of Tg(s) not bound to LCA related to the amount of total Tg(s) in sample from the papillary carcinoma is significantly higher than those in the sample from benign disease such as follicular adenoma, thyroid adnomatous, Grave's disease.

Namely, the method of determining malignancy of thyroid tumor of the present invention has been accomplished on the basis of those facts which have been found for the first time by the present inventors.

The reagent for measurement of Tg(s) of the present invention comprises each one or more of "proteins capable of binding to a constant region of Tg(s)" and"proteins capable of specifically binding to a specific sugar chain structure of Tg(s) having the specific sugar chain structure" , and the preferable embodiments and specific examples thereof are as mentioned above.

The reagent for determination of malignancy of thyroid adenoma comprises each one or more of "proteins capable of binding to a constant region of Tg(s)" and "proteins capable of specifically binding to a specific sugar chain structure of Tg(s) having the specific sugar chain structure", and the preferable embodiments and specific examples

thereof are as mentioned above.

In the reagents, there may be incorporated reagents having generally been used in this field so far as not interfering stability of co-existing reagents and not interfering the reaction of Tg(s) with "Tg-binding proteins" and/or "proteins binding to a specific sugar chain structure" , such as a buffering agent, a reaction accelerator and a stabilizer such as a sugar, a protein, a salt and a surfactant, antiseptic, etc., and their contents may be selected from a range having been generally used in this field.

Further, a metal ion such as magnesium has been known as not influencing upon activity and stability of lectin, and thus the metal ion may also be incorporated in the reagents.

The buffering agent usable in the reagents of the present invention includes all ones having been generally used in immunoturbidmetry, immunonephelometry, RIA, EIA, etc. such as Tris buffer, phosphate buffer, veronal buffer, borate buffer and Good's buffer, and pH upon measurement is not specifically limited so far as an antibody-antigen reaction, a reaction of Tg(s) with lectin, etc. are not restrained and generally 6 to 10.

In the following, the present invention is further explained in detail, and the present invention is not limited thereto by any means.

Example 1 A method for differentiation of papillary tumors using

Concanavalin A (ConA)

(Peroxidase labeled anti Tg antibody)

An anti Tg antibody (hereinafter abbreviated as "anti Tg-1") was bound to peroxidase after a conventional manner to give peroxidase labeled anti Tg antibody (hereinafter abbreviated as "anti Tg-1·POD").

(Sample)

Human thyroid tissue pieces in an amount of 0.1 g (wet wt) were homogenized in 1 ml of 0.1 M phosphate buffer solution (pH 7.5, containing 0.9 % NaCl, hereinafter abbreviated as "PBS") by a homogenizer, followed by centrifuging at 4°C at 30000 g for 5 minutes, and thus obtained supernatant was used as the sample.

The human thyroid tissues used were 11 papillary carcinoma specimens, 5 benign thyroid adenoma specimens, 5 Graves' disease specimens and 5 normal thyroid tissue specimens.

(Reagent 1)

Con A (manufactured and sold by Honen Corp.) was dissolved in PBS to give 15 mg/ml solution, which was used as Reagent 1.

(Pre-treatment of Sample)

Each 50 μl of Sample and Reagent 1 were mixed with each other, incubated at 4°C for overnight and centrifuged at 4°C at 3000g for 20 min. to remove precipitates, and the resulting supernatant was used as Pre-treatment solution 1. By the way, Tg(s) bound to Con A was removed by the centrifugation.

PBS was used in place of Reagent 1 after a similar manner to give supernatant which was used as Pre-treatment solution 2.

(Measurement of Tg content)

PBS in an amount of 1 ml containing 5 μg of an anti Tg antibody having an epitope different from that of "anti Tg-1" (hereinafter abbreviated as "anti Tg-2") was charged in each of 96 wells of a microplate, followed by keeping standing at 20°C for 1 hour and washing with PBS, whereby "anti Tg-2" was immobilized. Then, 0.2 ml of PBS containing 1 % of fetal bovine serum was added to each of the wells, followed by keeping standing at 20°C for 1 hour and washing with PBS, whereby blocking treatment was attained. PBS in an amount of 100 μl containing 1 % of fetal bovine serum and 50 μl of "Pre-treatment solution 1" or "Pre-treatment solution 2" were charged to each of the wells, followed by allowing a reaction to take place at 20°C for 1 hour. After the reaction, each of the wells was washed with PBS, and 100 μl of "anti Tg-1·POD" diluted 10000 times was added thereto, followed by allowing a reaction to take place at 20°C for 1 hour. After the reaction, the wells were washed with PBS and 100 μl of 3,3',5,5'-tetramethylbenzidine solution (manufactured and sold by Kirkegaad and Perry Labs. Inc.) was added thereto, followed by allowing a reaction to take place at 20°C for 30 minutes and terminating the reaction by addition of 50 μl of 1 M phosphoric acid. Absorbances at 450

nm of each of wells were measured by Microplate reader Spectra 1 (manufactured and sold by Wako Pure Chemical Industries, Ltd.) and the measured absorbances were applied to a calibration curve showing a relationship between Tg (s) content and absorbance which was previously prepared by using a Tg(s) solution containing a known amount of Tg (s) after a similar manner, whereby Tg(s) contents in "Pre-treating solution 1" and "Pre-treating solution 2" .

Tg(s) content in "Pre-treating solution 1" shows a content of Tg(s) not bound to Con A, and Tg(s) content in "Pre-treating solution 2" shows a total Tg(s) content.

(Calculation of a ratio of Tg(s) not bound to Con A)

A ratio (%) of Tg(s) not bound to Con A is calculated by the following equation.

$$\text{Ratio (\%) of Tg(s) not bound to Con A} = [(\text{an amount of Tg(s) not bound to Con A}) / (\text{total Tg(s)})] \times 100$$

(Result)

Thus obtained ratio (%) of Tg(s) not bound to Con A is shown in Figure 1.

As is clear from Figure 1, a ratio (%) of Tg(s) not bound to Con A in the extract from tissue of papillary carcinoma is significantly higher than those in the extracts from benign thyroid adenoma tissue, Graves' disease tissue and normal thyroid tissue. It is also understood that no difference in a ratio (%) of Tg(s) not bound to Con A is found among benign thyroid adenoma tissue extract, Graves' disease tissue extract and normal thyroid tissue extract. Namely, it can be recognized that an amount of Tg whose sugar chain is changed, in other words, Tg(s) not capable of binding to Con A is increased in papillary carcinoma tissue, and thus a ratio (%) of Tg(s) not bound to Con A is very useful for differentiation of benign thyroid adenoma from papillary carcinoma.

Example 2 A method for differentiation of papillary carcinoma using *Ricinus communis* agglutinin-120 (RCA120)

(Peroxidase labeled anti Tg antibody)

Same as Example 1

(Sample)

Sample was prepared by the same manner as in Example 1. The human thyroid tissues used were 7 papillary carcinoma specimens, 5 Graves' disease specimens and 4 normal thyroid tissue specimens.

(Reagent 1)

RCA120 (manufactured and sold by Honen Corp.) was dissolved in PBS to give 2.5 mg/ml solution, which was used as Reagent 1.

(Pre-treatment of Sample)

Pre-treatment solution 1 and 2 were prepared by the same manner as in Example 1, except for using Reagent 1 containing RCA 120 instead of Reagent 1 containing ConA.

(Measurement of Tg content)

Measurement of Tg(s) content was conducted after the same manner as in Example 1.

Tg(s) content in Pre-treatment solution 1 shows a content of Tg(s) not bound to RCA120 and that in Pre-treatment solution 2 shows a total Tg(s) content.

(Calculation of a ratio of Tg(s) not bound to RCA120)

A ratio (%) of Tg(s) not bound to RCA120 is calculated by the following equation.

$$\text{Ratio (\%) of Tg(s) not bound to RCA120} = [(\text{an amount of Tg(s) not bound to RCA120} / (\text{total Tg(s)})] \times 100$$

(Result)

Thus obtained ratio (%) of Tg(s) not bound to RCA120 is shown in Figure 2.

As is clear from Figure 2, a ratio (%) of Tg(s) not bound to RCA120 in the extract of tissue of papillary carcinoma is significantly higher than those in the extracts of benign goiter tissue, Graves' disease tissue and normal thyroid tissue. Namely, it can be recognized that an amount of Tg(s) whose sugar chain is changed, in other words, Tg(s) not capable of binding to RCA120 is increased in papillary carcinoma tissue, and thus a ratio (%) of Tg(s) not bound to RCA120 is very useful for differentiation of papillary carcinoma.

Example 3 Differentiation of follicular carcinoma from follicular adenoma using Con A.

(Peroxidase labeled anti Tg antibody)

Same as in Example 1

(Sample)

Sample was prepared by the same manner as in Example 1. The human thyroid tissues used were 4 follicular carcinoma specimens, 7 follicular adenoma specimens and 5 normal thyroid tissue specimens.

(Reagent 1)

Same as in Example 1

(Pre-treatment of Sample)

Pre-treatment solution 1 and 2 were prepared by the same manner as in Example 1.

(Measurement of Tg(s) content)

Measurement of Tg(s) content was conducted by the same manner as in Example 1.

Tg(s) content in Pre-treatment solution 1 shows a content of Tg(s) not bound to Con A and that in Pre-treatment solution 2 shows a total Tg(s) amount.

(Calculation of a ratio of Tg(s) not bound to Con A)

A ratio (%) of Tg(s) not bound to Con A was calculated by the same manner as in Example 1.

(Result)

Thus obtained ratio (%) of Tg(s) not bound to Con A is shown in Figure 3.

As is clear from Figure 3, a ratio (%) of Tg(s) not bound to Con A becomes higher and higher from a normal thyroid tissue extract, then a follicular carcinoma tissue extract, finally to a follicular adenoma tissue extract in this order, and it can be understood that there is found a significant difference in the ratio between a follicular carcinoma tissue extract and a follicular adenoma tissue extract. From this result, it can be recognized that differentiation between follicular carcinoma and follicular adenoma, which has been difficult even by cytodiagnosis, can easily be attained by measuring a ratio (%) of Tg(s) not bound to Con A.

Example 4 Fractional measurement of Tg(s) having different sugar chain structures using *Lens culinaris* agglutinin (LCA)

(Peroxidase labeled anti Tg antibody Fab' fragment)

Two kinds of anti Tg antibodies having different recognizable epitopes from each other, which do not bind to Tg bound to LCA (hereinafter abbreviated as "anti Tg-86" and "anti Tg-78", respectively), were treated after a conventional manner to give Fab' fragments and then they were bound to peroxidase (manufactured and sold by Toyobo Co., Ltd.) after a conventional manner to give peroxidase labeled anti Tg antibody Fab' fragments (hereinafter abbreviated as "anti Tg-86·Fab'-POD" , "anti Tg-78·Fab'-POD", respectively).

(Antibody solution 1)

A 50 mM phosphate buffer solution (pH 7.5, containing 0.15M NaCl and 0.5 (w/v)% bovine serum albumin) containing 2 nM of "anti Tg-86·Fab'-POD" was prepared, which was used as Antibody solution 1.

(Antibody solution 2)

A 50 mM phosphate buffer solution (pH 7.5, containing 0.15M NaCl and 0.5 (w/v)% bovine serum albumin) containing 3 nM of "anti Tg-78·Fab'-POD" was prepared, which was used as Antibody solution 2.

(Reagent 1)

A 50 mM phosphate buffer solution (pH 7.5, 0.15 M NaCl) containing 15 μM of LCA (manufactured and sold by Honen Corp.) was prepared, which was used as Reagent 1.

(Sample)

Human thyroid tissue pieces in an amount of 0.1 g (wet wt) were homogenized in 1 ml of 0.1 M phosphate buffer (pH 7.2, containing 0.9 % of NaCl) using a homogenizer, followed by centrifuging at 4°C at 100000 g for 60 minutes, and the supernatant obtained was diluted 200 to 1100 times with 50 mM phosphate buffer (pH 7.5, containing 0.15 M NaCl and 0.5 (w/v) % of bovine serum albumin) to give the sample.

The human thyroid tissues used were 4 benign disease specimens (1 follicular adenoma specimen, 2 adenomotous specimens and 1 Graves' disease) and 4 papillary carcinoma specimens.

(Measurement method)

Sample in an amount of 25 μl was mixed with 15 μl of Reagent 1, followed by allowing a reaction to take place at 8°C for 1 hour. The reaction solution in an amount of 15 μl was mixed with 90 μl of Antibody solution 1, followed by allowing a reaction to take place further at 8°C for 30 minutes. The resulting reaction solution in an amount of 50 μl was analyzed using high-performance liquid chromatography (HPLC) under the conditions as mentioned below to measure an amount of Tg(s) not bound to LCA. The same measurement for the total Tg(s) amount was conducted on the same sample using 50 mM of phosphate buffer (pH 7.5, containing 0.15M NaCl and 0.5 (w/v) % bovine serum albumin) in place of Reagent 1 containing LCA. Those two results were applied to the following equation to calculate a ratio (%) of Tg(s) not bound to LCA upon using "anti Tg-86·Fab'-POD".

$$\text{A ratio (\%) of Tg(s) not bound to LCA} = [(\text{an amount of Tg(s) not bound to LCA}) / (\text{total Tg(s)})] \times 100$$

A ratio (%) of Tg(s) not bound to LCA upon using "anti Tg-78·Fab'-POD" was also calculated by the same manner except for using Antibody solution 2 in place of Antibody solution 1.

(HPLC conditions)

Column: Wakopack Wakosil® -5Diol-200 8.0 mm × 300 mm (w)
Eluent: 50 mM phosphate buffer, pH 7.5, 0.15 M NaCl
Substrate solution: 15 mM citrate buffer (pH 5.5, 313 mM acetoaminophenol (manufactured and sold by Dojin Laboratories, containing 0.12 % $H_2O_2$)
Flow rate: the eluent 1.0 mL/min, the substrate solution 0.1 mL/min Detection: Ex 328 nm, Em 432 nm

(Result)

Ratios (%) of Tg(s) not bound to LCA upon using "anti Tg-86·Fab'-POD" and "anti Tg-78·Fab'-POD" are shown in Figure 4 and Figure 5, respectively.

It is understood from Figure 4 and Figure 5 that the ratio (%) of Tg(s) not bound to LCA in a papillary carcinoma tissue extract is significantly higher than that in a benign disease tissue extract.

Namely, it is understood that differentiation diagnosis of papillary carcinoma from benign disease becomes possible by using LCA.

Example 5 Fractional measurement of Tg (s) having different sugar chain structures using Con A

(Peroxidase labeled anti Tg antibody Fab' fragment)

"Anti Tg-86·Fab'-POD" prepared in Example 4 was used.

(Antibody solution 1)

A 50 mM phosphate buffer solution (pH 7.5, containing 0.15 M NaCl and 0.5 (w/v) % bovine serum albumin) containing 4 nM of "anti Tg-86·Fab'-POD" was prepared which was used as Antibody solution 1.

(Reagent 1)

A 50 mM phosphate buffer solution pH 7.5, 0.15 M NaCl, containing 15 μM of Con A (manufactured and sold by Honen Corp.) was prepared which was used as Reagent 1.

(Sample)

The same one as in Example 4 was used.

(Measurement method)

Sample in an amount of 10 μl was mixed with 45 μl of Reagent 1, followed by allowing a reaction to take place at 8°C for 1 hour. The reaction solution was mixed with 45 μl of Antibody solution 1, following by allowing a reaction to take place further at 8°C for 30 minutes. The resulting reaction solution in an amount of 50 μl was analyzed using high-performance liquid chromatography (HPLC) under the conditions as mentioned below to measure an amount of Tg(s) not bound to Con A. The same measurement for the total Tg(s) amount was conducted on the same sample using 50 mM of phosphate buffer (pH 7.5, containing 0.15M NaCl and 0.5 (w/v) % bovine serum albumin) in place of Reagent 1. Those two results were applied to the following equation to calculate a ratio (%) of Tg(s) not bound to Con A.

$$\text{A ratio (\%) of Tg(s) not bound to Con A} = [(\text{an amount of Tg(s) not bound to Con A}) / (\text{total Tg(s)})] \times 100$$

(HPLC conditions)

Same as in Example 4

(Result)

The obtained ratio (%) of Tg(s) not bound to Con A is shown in Figure 6.

It is understood from Figure 6 that a ratio of Tg(s) not bound to Con A in papillary carcinoma tissue extract is significantly higher than that in a benign disease tissue extract.

Namely, it is understood that differentiation diagnosis of papillary carcinoma from benign disease becomes possible by using Con A.

The present invention is to provide a method for measuring simply and at high accuracy various kinds of Tg (s) in various kinds living samples and a reagent for this method, and differentiation of papillary carcinoma from benign thyroid adenoma and differentiation of follicular carcinoma from follicular adenoma can be conducted by using a suitable combination of the measurement results of various kinds of Tg(s).

## Claims

**1.** A method of determining a malignancy of thyroid tumor, which comprises

(A) measuring the amount of a thyroglobulin (Tg) having a specific sugar chain structure, wherein the specific sugar chain structure is a sugar chain structure of Tg capable of binding to a lectin selected from Concanavalin A, *Lens culinaris* agglutinin and *Ricinus communis* agglutinin, and/or of a Tg having a sugar chain structure other than said specific sugar chain structure by any one of (1) to (9) as mentioned below:

(1):

(i) reacting the sample with either one of (a) protein-(1) which is an anti-Tg antibody capable of binding to a constant region of Tg or a receptor capable of binding to Tg, or (b) a protein-(2) which is an antibody or a lectin capable of specifically binding to said specific sugar chain structure of Tg, to form a conjugate of [protein (1)]-[Tg] or [protein-(2)]-[Tg having said specific sugar chain structure],
(ii) reacting the resulting conjugate with the other one of (a) the protein-(1) or (b) the protein-(2), which is labeled by a labeling substance, to form a conjugate of [protein-(1)]-[Tg having said specific sugar chain structure]-[labeled protein-(2)] or [protein-(2)]-[Tg having said specific sugar chain structure]-[labeled protein-(1)],
(iii) separating the conjugate from the free labeled protein-(2) or the free labeled protein-(1), and
(iv) measuring the amount of the labeling substance in the conjugate formed in step (ii), whereby the amount of Tg having said specific sugar chain structure is obtained;

(2):

(i) reacting the sample with a labeled protein-(2), which is an antibody or a lectin capable of specifically binding to said specific sugar chain structure of Tg, and a protein-(3), which is an anti-Tg antibody or a receptor capable of binding to all Tg regardless whether the protein-(2) is already bound thereto or not, to form a conjugate of [labeled protein-(2)]-[Tg having said specific sugar chain structure]-[protein-(3)],
(ii) separating the conjugate from the free labeled protein-(2), and
(iii) measuring the amount of the labeling substance in the conjugate formed in step (i), whereby the amount of Tg having said specific sugar chain structure is obtained;

(3):

(i) reacting the sample with a protein-(2) which is an antibody or a lectin capable of specifically binding to said specific sugar chain structure of Tg, to form a conjugate of [Tg having said specific sugar chain structure]-[protein-(2)],
(ii) separating the conjugate from Tg having a sugar chain structure other than said specific sugar chain structure to which the protein-(2) is not bound, and
(iii) measuring the amount of Tg having said specific sugar chain structure in the conjugate or the amount of Tg having a sugar chain structure other than said specific sugar chain structure;

(4):

(i) reacting the sample with a protein-(1), which is an anti-Tg antibody capable of binding to a constant region of Tg or a receptor capable of binding to Tg, to form a conjugate of [protein-(1)]-[Tg],

(ii) reacting the conjugate with both of a protein-(2), which is an antibody or a lectin capable of specifically binding to said specific sugar chain structure of Tg, and a labeled protein-(4), which is an anti-Tg antibody or a receptor capable of binding to Tg but not capable of binding to Tg to which the protein-(2) is already bound, to form a conjugate-(a) of [protein-(1)]-[Tg having said specific sugar chain structure]-[protein-(2)] and a conjugate-(b) of [protein-(1)]-[Tg having a sugar chain structure other than said specific sugar chain structure]-[labeled protein-(4)],
(iii) separating the conjugates from the free labeled protein-(4), and
(iv) measuring the amount of the labeling substance in the conjugate-(b) formed in step (ii), whereby the amount of Tg having a sugar chain structure other than said specific sugar chain structure is obtained;

(5):

(i) reacting the sample with a protein-(2) which is an antibody or a lectin capable of specifically binding to said specific sugar chain structure of Tg, and then with a protein-(4), which is an anti-Tg antibody or a receptor capable of binding to Tg but not capable of binding to Tg to which the protein-(2) is already bound, to form a conjugate-(a) of [protein-(2)]-[Tg having said specific sugar chain structure] and a conjugate-(b) of [protein-(4)]-[Tg having a sugar chain structure other than said specific sugar chain structure],
(ii) separating the conjugate-(b) from others,
(iii) reacting the conjutate-(b) with a labeled protein-(1), which is an anti-Tg antibody capable of binding to a constant region of Tg or a receptor capable of binding to Tg, to form a conjugate-(c) of [protein-(4)]-[Tg having the sugar chain structure other than said specific sugar chain structure]-[labeled protein-(1)],
(iv) separating the conjugate-(c) from free labeled protein-(1), and
(v) measuring the amount of the labeling substance in the conjugate-(c) formed in step (iii), whereby the amount of Tg having a sugar chain structure other than said specific sugar chain structure is obtained;

(6):

(i) reacting a sample with both a protein-(2), which is an antibody or a lectin capable of specifically binding to said specific sugar chain structure of Tg, and a labeled protein-(4), which is an anti-Tg antibody or a receptor capable of binding to Tg but not capable of binding to Tg to which the protein-(2) is already bound, to form a conjugate-(a) of [protein-(2)]-[Tg having said specific sugar chain structure] and a conjugate-(b) of [labeled protein-(4)]-[Tg having a sugar chain structure other than said specific sugar chain structure],
(ii) separating the conjugate-(b) from the free labeled protein-(4), and
(iii) measuring the amount of the labeling substance in the conjugate-(b) formed in step (ii), whereby the amount of Tg having a sugar chain structure other than said specific sugar chain structure is obtained;

(7):

(i) reacting the sample with both of a labeled protein-(1), which is an anti-Tg antibody capable of binding to a constant region of Tg or a receptor capable of binding to Tg, and a protein-(2), which is an antibody or a lectin capable of specifically binding to said specific sugar chain structure of Tg, to form a conjugate-(a) of [labeled protein-(1)]-[Tg having said specific sugar chain structure]-[protein-(2)] and a conjugate-(b) of [labeled protein-(1)]-[Tg having a sugar chain structure other than said specific sugar chain structure],
(ii) separating the each conjugates from the free labeled protein-(1), and
(iii) measuring the amounts of the labeling substances in each of the conjugate-(a) and conjugate-(b) formed in step (i), respectively, whereby amounts of Tg having said specific sugar chain structure, Tg having a sugar chain structure other than said specific sugar chain structure and total Tg are obtained;

(8):

(i) reacting the sample with a protein-(2), which is an antibody or a lectin capable of specifically binding to said specific sugar chain structure of Tg, and a labeled protein-(3), which is an anti-Tg antibody or a receptor capable of binding to all Tg regardless whether the protein-(2) is already bound thereto or not, and a protein-(4), which is an anti-Tg antibody or a receptor capable of binding to Tg but not capable

of binding to Tg to which the protein-(2) is already bound, to form a conjugate-(a) of [labeled protein-(3)]-[Tg having said specific sugar chain structure]-[protein-(2)] and a conjugate-(b) of [labeled protein-(3)]-[Tg having a sugar chain structure other than said specific sugar chain structure]-[protein-(4)],
(ii) removing the free labeled protein-(3), and separating the conjugate-(a) and (b), and
(iii) measuring the amounts of the labeling substance in each conjugate-(a) and (b) formed in step (i), whereby amounts of Tg having said specific sugar chain structure, Tg having a sugar chain structure other than said specific sugar chain structure and total Tg are obtained;

(9):

(i) reacting the sample with a labeled protein-(1), which is an anti-Tg antibody capable of binding to a constant region of Tg or a receptor capable of binding to Tg, and a protein-(2), which is an antibody or a lectin capable of specifically binding to said specific sugar chain structure of Tg, and a protein-(4), which is an anti-Tg antibody or a receptor capable of binding to Tg but not capable of binding to Tg to which the protein-(2) is already bound, to form a conjugate-(a) of [labeled protein-(1)]-[Tg having said specific sugar chain structure]-[protein-(2)] and a conjugate-(b) of [labeled protein-(1)]-[Tg having a sugar chain structure other than said specific sugar chain structure]-[protein-(4)],
(ii) removing the free labeled protein-(1), and separating the conjugate-(a) and (b), and
(iii) measuring the amounts of the labeling substances in each conjugate-(a) and (b) formed in step (i), whereby amounts of Tg having said specific sugar chain structure, Tg having a sugar chain structure other than said specific sugar chain structure and total Tg are obtained;

(B) measuring the amount of total Tg by any one of the method i) to ii) below:

(i) an immunological assay using a Tg binding protein, and
(ii) (7) to (9) as defined in (A);

(C) calculating the ratio of the amount of the Tg having said specific sugar chain structure present in a sample to the amount of total Tg, or the ratio of the amount of the Tg having a sugar chain structure other than said specific sugar chain structure to the amount of total Tg; and
(D) determining the malignancy of the thyroid tumor by comparing the calculated ratio with a corresponding predetermined ratio from a reference fluid sample originating from a living body having a normal thyroid or a benign thyroid,

wherein the sample is determined to be malignant when the calculated ratio is significantly different from that of the reference fluid sample of the normal thyroid and is significantly different from that of the reference fluid sample of the benign thyroid, or when the calculated ratio is significantly different from that of the reference fluid sample of the benign thyroid and is not significantly different from that of the reference fluid sample of the normal thyroid.

**2.** The method according to claim 1, wherein the lectin is capable of binding to D-galactose or N-acetyl-D-galactosamine, or binding to D-mannose.

**3.** The method according to claim 1, wherein the sugar chain structure not capable of binding to the lectin is found in thyroglobulin(s) which is produced by a carcinoma cell.

**4.** The method as claimed in claim 3, wherein the carcinoma cell is originated from thyroid carcinoma.

**5.** A use of (a) an anti-Tg antibody capable of binding to a constant region of Tg and/or a receptor capable of binding to Tg, and (b) an antibody and/or a lectin capable of specifically binding to a specific sugar chain structure of Tg, wherein the specific sugar chain structure is a sugar chain structure of Tg capable of binding to a lectin selected from Concanavalin A, *Lens culinaris* agglutinin and *Ricinus communis* agglutinin, in a method for determining a malignancy of a thyroid tumor as defined in claim 1.

**6.** A reagent for determining a malignancy of thyroid tumor, which comprises:

(1) an anti-Tg antibody capable of binding to a constant region of Tg and optionally a receptor capable of binding to Tg, and

(2) a lectin or an antibody capable of binding to a specific sugar chain structure, wherein the specific sugar chain structure is a sugar chain structure of Tg capable of binding to a lectin selected from Concanavalin A, *Lens culinaris* agglutinin and *Ricinus communis* agglutinin.

**Patentansprüche**

1. Verfahren zur Bestimmung der Malignität eines Schilddrüsentumors, umfassend:

(A) das Messen der Menge eines Thyroglobulins (Tg) mit einer spezifischen Zuckerkettenstruktur, wobei es sich bei der spezifischen Zuckerkettenstruktur um eine Zuckerkettenstruktur eines Tg, das an ein Lectin binden kann, das aus Concanavalin A, *Lens-culinaris*-Agglutinin und *Ricinus-communis*-Agglutinin ausgewählt ist, und/oder eines Tg, das eine andere Zuckerkettenstruktur als die spezifische Zuckerkettenstruktur aufweist, handelt, durch eines der folgenden Verfahren (1) bis (9):

(1):

(i) Umsetzen der Probe mit entweder (a) Protein-(1), bei dem es sich um einen Anti-Tg-Antikörper, der an einen konstanten Bereich von Tg binden kann, oder einen Rezeptor, der an Tg binden kann, handelt, oder (b) einem Protein-(2), bei dem es sich um einen Antikörper oder ein Lectin handelt, der bzw. das spezifisch an die spezifische Zuckerkettenstruktur des Tg binden kann, unter Bildung eines Konjugats aus [Protein-(1)]-[Tg] oder [Protein-(2)]-[Tg mit der spezifischen Zuckerkettenstruktur];
(ii) Umsetzen des resultierenden Konjugats mit dem jeweils anderen von (a) dem Protein-(1) oder (b) dem Protein-(2), das mit einer Markersubstanz markiert ist, unter Bildung eines Konjugats aus [Protein-(1)]-[Tg mit der spezifischen Zuckerkettenstruktur]-[markiertem Protein-(2)] oder [Protein-(2)]-[Tg mit der spezifischen Zukkerkettenstruktur]-[markiertem Protein-(1)];
(iii) Abtrennen des Konjugats von dem freien markierten Protein-(2) oder dem freien markierten Protein-(1); und
(iv) Messen der Menge der Markersubstanz in dem in Schritt (ii) gebildeten Konjugat, wodurch die Menge des Tg mit der spezifischen Zuckerkettenstruktur erhalten wird;

(2):

(i) Umsetzen der Probe mit einem markierten Protein-(2), bei dem es sich um einen Antikörper oder ein Lectin handelt, der bzw. das spezifisch an die spezifische Zuckerkettenstruktur des Tg binden kann, und einem Protein-(3), bei dem es sich um einen Anti-Tg-Antikörper oder einen Rezeptor handelt, der an alle Tg unabhängig davon, ob das Protein-(2) bereits daran gebunden ist oder nicht, binden kann, unter Bildung eines Konjugats aus [markiertem Protein-(2)]-[Tg mit der spezifischen Zuckerkettenstruktur]-[Protein-(3)];
(ii) Abtrennen des Konjugats von dem freien markierten Protein-(2); und
(iii) Messen der Menge der Markersubstanz in dem in Schritt (i) gebildeten Konjugat, wodurch die Menge des Tg mit der spezifischen Zuckerkettenstruktur erhalten wird;

(3):

(i) Umsetzen der Probe mit einem Protein-(2), bei dem es sich um einen Antikörper oder ein Lectin handelt, der bzw. das spezifisch an die spezifische Zuckerkettenstruktur des Tg binden kann, unter Bildung eines Konjugats aus [Tg mit der spezifischen Zuckerkettenstruktur]-[Protein-(2)];
(ii) Abtrennen des Konjugats von dem Tg, das eine andere Zuckerkettenstruktur als die spezifische Zuckerkettenstruktur aufweist, an die das Protein-(2) nicht gebunden ist; und
(iii) Messen der Menge des Tg mit der spezifischen Zuckerkettenstruktur im Konjugat oder der Menge des Tg, das eine andere Zuckerkettenstruktur als die spezifische Zuckerkettenstruktur aufweist;

(4):

(i) Umsetzen der Probe mit einem Protein-(1), bei dem es sich um einen Anti-Tg-Antikörper, der an einen konstanten Bereich von Tg binden kann, oder einen Rezeptor, der an Tg binden kann, handelt, unter Bildung eines Konjugats aus [Protein-(1)]-[Tg];

(ii) Umsetzen des Konjugats mit sowohl einem Protein-(2), bei dem es sich um einen Antikörper oder ein Lectin handelt, der bzw. das spezifisch an die spezifische Zuckerkettenstruktur des Tg binden kann, als auch einem markierten Protein-(4), bei dem es sich um einen Anti-Tg-Antikörper oder einen Rezeptor, der an Tg binden kann, aber nicht an Tg, an das das Protein-(2) bereits gebunden ist, binden kann, handelt, unter Bildung eines Konjugats-(a) aus [Protein-(1)]-[Tg mit der spezifischen Zuckerkettenstruktur]-[Protein-(2)] und eines Konjugats-(b) aus [Protein-(1)]-[Tg mit einer anderen Zuckerkettenstruktur als der spezifischen Zuckerkettenstruktur]-[markiertem Protein-(4)];
(iii) Abtrennen des Konjugats von dem freien markierten Protein-(4); und
(iv) Messen der Menge der Markersubstanz in dem in Schritt (ii) gebildeten Konjugat-(b), wodurch die Menge des Tg mit einer anderen Zuckerkettenstruktur als der spezifischen Zuckerkettenstruktur erhalten wird;

(5):

(i) Umsetzen der Probe mit einem Protein-(2), bei dem es sich um einen Antikörper oder ein Lectin handelt, der bzw. das spezifisch an die spezifische Zuckerkettenstruktur des Tg binden kann, und dann mit einem Protein-(4), bei dem es sich um einen Anti-Tg-Antikörper oder einen Rezeptor, der an Tg binden kann, aber nicht an Tg, an das das Protein-(2) bereits gebunden ist, binden kann, handelt, unter Bildung eines Konjugats-(a) aus [Protein-(2)]-[Tg mit der spezifischen Zuckerkettenstruktur] und eines Konjugats-(b) aus [Protein-(4)]-[Tg mit einer anderen Zuckerkettenstruktur als der spezifischen Zukkerkettenstruktur];
(ii) Abtrennen des Konjugats-(b) von anderen;
(iii) Umsetzen des Konjugats-(b) mit einem markierten Protein-(1), bei dem es sich um einen Anti-Tg-Antikörper, der an einen konstanten Bereich von Tg binden kann, oder einen Rezeptor, der an Tg binden kann, handelt, unter Bildung eines Konjugats-(c) aus [Protein-(4)]-[Tg mit einer anderen Zukkerkettenstruktur als der spezifischen Zukkerkettenstruktur]-[markiertem Protein-(1)];
(iv) Abtrennen des Konjugats-(c) von freiem markiertem Protein-(1); und
(v) Messen der Menge der Markersubstanz in dem in Schritt (iii) gebildeten Konjugat-(c), wodurch die Menge des Tg mit einer anderen Zuckerkettenstruktur als der spezifischen Zuckerkettenstruktur erhalten wird;

(6):

(i) Umsetzen der Probe mit sowohl einem Protein-(2), bei dem es sich um einen Antikörper oder ein Lectin handelt, der bzw. das spezifisch an die spezifische Zuckerkettenstruktur des Tg binden kann, als auch einem markierten Protein-(4), bei dem es sich um einen Anti-Tg-Antikörper oder einen Rezeptor, der an Tg binden kann, aber nicht an Tg, an das das Protein-(2) bereits gebunden ist, binden kann, handelt, unter Bildung eines Konjugats-(a) aus [Protein-(2)]-[Tg mit der spezifischen Zuckerkettenstruktur] und eines Konjugats-(b) aus [markiertem Protein-(4)]-[Tg mit einer anderen Zuckerkettenstruktur als der spezifischen Zuckerkettenstruktur];
(ii) Abtrennen des Konjugats-(b) von dem freien markierten Protein-(4); und
(iii) Messen der Menge der Markersubstanz in dem in Schritt (ii) gebildeten Konjugat-(b), wodurch die Menge des Tg mit einer anderen Zuckerkettenstruktur als der spezifischen Zuckerkettenstruktur erhalten wird;

(7):

(i) Umsetzen der Probe mit sowohl einem markierten Protein-(1), bei dem es sich um einen Anti-Tg-Antikörper, der an einen konstanten Bereich von Tg binden kann, oder einen Rezeptor, der an Tg binden kann, handelt, als auch einem Protein-(2), bei dem es sich um einen Antikörper oder ein Lectin handelt, der bzw. das spezifisch an die spezifische Zuckerkettenstruktur des Tg binden kann, unter Bildung eines Konjugats-(a) aus [markiertem Protein-(1)]-[Tg mit der spezifischen Zuckerkettenstruktur]-[Protein-(2)] und eines Konjugats-(b) aus [markiertem Protein-(1)]-[Tg mit einer anderen Zuckerkettenstruktur als der spezifischen Zuckerkettenstruktur];
(ii) Abtrennen der jeweiligen Konjugate von dem freien markierten Protein-(1); und
(iii) Messen der Mengen der Markersubstanzen jeweils in dem in Schritt (i) gebildeten Konjugat-(a) und Konjugat-(b), wodurch die Mengen des Tg mit der spezifischen Zuckerkettenstruktur, des Tg mit einer anderen Zuckerkettenstruktur als der spezifischen Zuckerkettenstruktur und des Gesamt-Tg erhalten werden;

(8):

(i) Umsetzen der Probe mit einem Protein-(2), bei dem es sich um einen Antikörper oder ein Lectin handelt, der bzw. das spezifisch an die spezifische Zuckerkettenstruktur des Tg binden kann, und einem markierten Protein-(3), bei dem es sich um einen Anti-Tg-Antikörper oder einen Rezeptor handelt, der an alle Tg unabhängig davon, ob das Protein-(2) bereits daran gebunden ist oder nicht, binden kann, und einem Protein-(4), bei dem es sich um einen Anti-Tg-Antikörper oder einen Rezeptor, der an Tg binden kann, aber nicht an Tg, an das das Protein-(2) bereits gebunden ist, binden kann, handelt, unter Bildung eines Konjugats-(a) aus [markiertem Protein-(3)]-[Tg mit der spezifischen Zukkerkettenstruktur]-[Protein-(2)] und eines Konjugats-(b) aus [markiertem Protein-(3)]-[Tg mit einer anderen Zukkerkettenstruktur als der spezifischen Zuckerkettenstruktur]-[Protein-(4)];
(ii) Entfernen des freien markierten Proteins-(3) und Abtrennen des Konjugats-(a) und des Konjugats-(b); und
(iii) Messen der Mengen der Markersubstanzen jeweils in dem in Schritt (i) gebildeten Konjugat-(a) und Konjugat-(b), wodurch die Mengen des Tg mit der spezifischen Zuckerkettenstruktur, des Tg mit einer anderen Zuckerkettenstruktur als der spezifischen Zuckerkettenstruktur und des Gesamt-Tg erhalten werden;

(9):

(i) Umsetzen der Probe mit einem markierten Protein-(1), bei dem es sich um einen Anti-Tg-Antikörper, der an einen konstanten Bereich von Tg binden kann, oder einen Rezeptor, der an Tg binden kann, handelt, und einem Protein-(2), bei dem es sich um einen Antikörper oder ein Lectin handelt, der bzw. das spezifisch an die spezifische Zuckerkettenstruktur des Tg binden kann, und einem Protein-(4), bei dem es sich um einen Anti-Tg-Antikörper oder einen Rezeptor, der an Tg binden kann, aber nicht an Tg, an das das Protein-(2) bereits gebunden ist, binden kann, handelt, unter Bildung eines Konjugats-(a) aus [markiertem Protein-(1)]-[Tg mit der spezifischen Zuckerkettenstruktur]-[Protein-(2)] und eines Konjugats-(b) aus [markiertem Protein-(1)]-[Tg mit einer anderen Zuckerkettenstruktur als der spezifischen Zuckerkettenstruktur]-[Protein-(4)];
(ii) Entfernen des freien markierten Proteins-(1) und Abtrennen des Konjugats-(a) und des Konjugats-(b); und
(iii) Messen der Mengen der Markersubstanzen jeweils in dem in Schritt (i) gebildeten Konjugat-(a) und Konjugat-(b), wodurch die Mengen des Tg mit der spezifischen Zuckerkettenstruktur, des Tg mit einer anderen Zuckerkettenstruktur als der spezifischen Zuckerkettenstruktur und des Gesamt-Tg erhalten werden;

(B) das Messen des Gesamt-Tg nach einem der folgenden Verfahren i) bis ii):

(i) einem immunologischen Assay unter Verwendung eines Tgbindenden Proteins; und
(ii) (7) bis (9), wie sie unter (A) definiert sind;

(C) das Berechnen des Verhältnisses der in der Probe vorhandenen Menge des Tg mit der spezifischen Zukkerkettenstruktur zur Menge des Gesamt-Tg oder des Verhältnisses der Menge des Tg mit einer anderen Zuckerkettenstruktur als der spezifischen Zuckerkettenstruktur zur Menge des Gesamt-Tg; und
(D) das Bestimmen der Malignität des Schilddrüsentumors durch vergleichen des berechneten Verhältnisses mit einem entsprechenden vorbestimmten Verhältnis aus einer Referenzflüssigkeitsprobe, die von einem lebenden Körper stammt, der eine normale Schilddrüse oder eine gutartige Schilddrüse hat;

wobei die Probe als maligne bestimmt wird, wenn das berechnete Verhältnis signifikant von dem der Referenzflüssigkeitsprobe der normalen Schilddrüse abweicht und signifikant von dem der Referenzflüssigkeitsprobe der gutartigen Schilddrüse abweicht oder wenn das berechnete Verhältnis signifikant von dem der Referenzflüssigkeitsprobe der gutartigen Schilddrüse abweicht und nicht signifikant von dem der Referenzflüssigkeitsprobe der normalen Schilddrüse abweicht.

2. Verfahren gemäß Anspruch 1, wobei das Lectin an D-Galactose oder N-Acetyl-D-galactosamin oder an D-Mannose binden kann.

3. Verfahren gemäß Anspruch 1, wobei die Zuckerkettenstruktur, die nicht an das Lectin binden kann, bei Thyroglobulinen vorkommen, die von einer Karzinomzelle produziert werden.

4. Verfahren gemäß Anspruch 3, wobei die Karzinomzelle aus einem Schilddrüsenkarzinom stammt.

5. Verwendung (a) eines Anti-Tg-Antikörpers, der an einen konstanten Bereich von Tg binden kann, und/oder eines Rezeptors, der an Tg binden kann, sowie (b) eines Antikörpers und/oder Lectins, der bzw. das spezifisch an eine spezifische Zuckerkettenstruktur von Tg binden kann, wobei die spezifische Zuckerkettenstruktur eine Zuckerkettenstruktur eines Tg ist, das an ein Lectin binden kann, das aus Concanavalin A, *Lens-culinaris*-Agglutinin und *Ricinus-communis*-Agglutinin ausgewählt ist, in einem Verfahren zur Bestimmung der Malignität eines Schilddrüsentumors, wie es in Anspruch 1 definiert ist.

6. Reagens zur Bestimmung der Malignität eines Schilddrüsentumors, umfassend:

(1) einen Anti-Tg-Antikörper, der an einen konstanten Bereich von Tg binden kann, und gegebenenfalls einen Rezeptor, der an Tg binden kann; und
(2) ein Lectin und/oder einen Antikörper, der bzw. das an eine spezifische Zuckerkettenstruktur binden kann, wobei die spezifische Zuckerkettenstruktur eine Zuckerkettenstruktur eines Tg ist, das an ein Lectin binden kann, das aus Concanavalin A, *Lens-culinaris*-Agglutinin und *Ricinus-communis*-Agglutinin ausgewählt ist.

**Revendications**

1. Procédé destiné à déterminer une malignité d'une tumeur de la thyroïde, qui comprend

(A) de mesurer la quantité d'une thyroglobuline (Tg) ayant une structure de chaîne glucidique spécifique, dans laquelle la structure de chaîne glucidique spécifique est une structure de chaîne glucidique d'une Tg capable de se lier à une lectine choisie parmi la concanavaline A, l'agglutinine de *Lens culinaris* et l'agglutinine de *Ricinus communis*, et/ou d'une Tg ayant une structure de chaîne glucidique autre que ladite structure de chaîne glucidique spécifique, par l'un quelconque des paragraphes (1) à (9) comme mentionnés ci-dessous :

(1) :

(i) faire réagir l'échantillon avec l'une parmi (a) une protéine-(1), qui est un anticorps anti-Tg capable de se lier à une région constante de Tg ou un récepteur capable de se lier à Tg, ou (b) une protéine-(2), qui est un anticorps ou une lectine capable de se lier spécifiquement à ladite structure de chaîne glucidique spécifique de Tg, afin de former un conjugué de [protéine-(1)]-[Tg] ou de [protéine-(2)]-[Tg ayant ladite structure de chaîne glucidique spécifique],
(ii) faire réagir le conjugué résultant avec l'autre parmi (a) la protéine-(1) ou (b) la protéine-(2), qui est marquée par une substance de marquage, afin de former un conjugué de [protéine-(1)]-[Tg ayant ladite structure de chaîne glucidique spécifique]-[protéine-(2) marquée] ou [protéine-(2)]-[Tg ayant ladite structure de chaîne glucidique spécifique]-[protéine-(1) marquée],
(iii) séparer le conjugué de la protéine (2) marquée libre ou de la protéine (1) marquée libre, et
(iv) mesurer la quantité de la substance de marquage dans le conjugué formé dans l'étape (ii), d'où il résulte l'obtention de la quantité de Tg ayant ladite structure de chaîne glucidique spécifique ;

(2) :

(i) faire réagir l'échantillon avec une protéine-(2) marquée, qui est un anticorps ou une lectine capable de se lier spécifiquement à ladite structure de chaîne glucidique spécifique de Tg, et une protéine-(3), qui est un anticorps anti-Tg ou un récepteur capable de se lier à toutes les Tg indépendamment du fait que la protéine-(2) y soit déjà liée ou non, afin de former un conjugué de [protéine- (2) marquée]-[Tg ayant ladite structure de chaîne glucidique spécifique]-[protéine-(3)],
(ii) séparer le conjugué de la protéine-(2) marquée libre , et
(iii) mesurer la quantité de la substance de marquage dans le conjugué formé dans l'étape (i), d'où il résulte l'obtention de la quantité de Tg ayant ladite structure de chaîne glucidique spécifique ;

(3) :

(i) faire réagir l'échantillon avec une protéine-(2), qui est un anticorps ou une lectine capable de se lier spécifiquement à ladite structure de chaîne glucidique spécifique de Tg, afin de former un conjugué de [Tg ayant ladite structure de chaîne glucidique spécifique]-[protéine-(2)],
(ii) séparer le conjugué de Tg ayant une structure de chaîne glucidique autre que ladite structure de chaîne glucidique spécifique à laquelle la protéine-(2) n'est pas liée, et
(iii) mesurer la quantité de Tg ayant ladite structure de chaîne glucidique spécifique dans le conjugué ou la quantité de Tg ayant une structure de chaîne glucidique autre que ladite structure de chaîne glucidique spécifique ;

(4) :

(i) faire réagir l'échantillon avec une protéine-(1), qui est un anticorps anti-Tg capable de se lier à une région constante de Tg ou un récepteur capable de se lier à Tg, afin de former un conjugué de [protéine-(1)]-[Tg],
(ii) faire réagir le conjugué à la fois avec une protéine-(2), qui est un anticorps ou une lectine capable de se lier spécifiquement à ladite structure de chaîne glucidique spécifique de Tg, et avec une protéine-(4) marquée, qui est un anticorps anti-Tg ou un récepteur capable de se lier à Tg mais incapable de se lier à Tg à laquelle la protéine-(2) est déjà liée, afin de former un conjugué (a) de [protéine-(1)]-[Tg ayant ladite structure de chaîne glucidique spécifique]-[protéine-(2)] et un conjugué (b) de [protéine-(1)]-[Tg ayant une structure de chaîne glucidique autre que ladite structure de chaîne glucidique spécifique]-[protéine-(4) marquée],
(iii) séparer les conjugués de la protéine-(4) marquée libre, et
(iv) mesurer la quantité de la substance de marquage dans le conjugué (b) formé dans l'étape (ii), d'où il résulte l'obtention de la quantité de Tg ayant une structure de chaîne glucidique autre que ladite structure de chaîne glucidique spécifique ;

(5) :

(i) faire réagir l'échantillon avec une protéine-(2), qui est un anticorps ou une lectine capable de se lier spécifiquement à ladite structure de chaîne glucidique spécifique de Tg, puis avec une protéine-(4), qui est un anticorps anti-Tg ou un récepteur capable de se lier à Tg mais incapable de se lier à Tg à laquelle la protéine-(2) est déjà liée, afin de former un conjugué (a) de [protéine-(2)]-[Tg ayant ladite structure de chaîne glucidique spécifique] et un conjugué (b) de [protéine-(4)]-[Tg ayant une structure de chaîne glucidique autre que ladite structure de chaîne glucidique spécifique],
(ii) séparer le conjugué (b) des autres,
(iii) faire réagir le conjugué (b) avec une protéine-(1) marquée qui est un anticorps anti-Tg capable de se lier à une région constante de Tg ou un récepteur capable de se lier à Tg, afin de former un conjugué (c) de [protéine-(4)]-[Tg ayant une structure de chaîne glucidique autre que ladite structure de chaîne glucidique spécifique]-[protéine-(1) marquée],
(iv) séparer le conjugué (c) de la protéine-(1) marquée libre, et
(v) mesurer la quantité de la substance de marquage dans le conjugué (c) formé dans l'étape (iii), d'où il résulte l'obtention de la quantité de Tg ayant une structure de chaîne glucidique autre que ladite structure de chaîne glucidique spécifique ;

(6) :

(i) faire réagir l'échantillon avec à la fois une protéine-(2), qui est un anticorps ou une lectine capable de se lier spécifiquement à ladite structure de chaîne glucidique spécifique de Tg, et une protéine-(4) marquée, qui est un anticorps anti-Tg ou un récepteur capable de se lier à Tg mais incapable de se lier à Tg à laquelle la protéine-(2) est déjà liée, afin de former un conjugué (a) de [protéine-(2)]-[Tg ayant ladite structure de chaîne glucidique spécifique] et un conjugué (b) de [protéine- (4) marquée]-[Tg ayant une structure de chaîne glucidique autre que ladite structure de chaîne glucidique spécifique], et
(ii) séparer le conjugué (b) de la protéine-(4) marquée libre, et
(iii) mesurer la quantité de la substance de marquage dans le conjugué (b) formé dans l'étape (iii), d'où il résulte l'obtention de la quantité de Tg ayant une structure de chaîne glucidique autre que ladite structure de chaîne glucidique spécifique ;

(7) :

(i) faire réagir l'échantillon avec à la fois une protéine-(1) marquée, qui est un anticorps anti-Tg capable de se lier à une région constante de Tg ou un récepteur capable de se lier à Tg, et une protéine-(2), qui est un anticorps ou une lectine capable de se lier spécifiquement à ladite structure de chaîne glucidique spécifique de Tg, afin de former un conjugué (a) de [protéine-(1) marquée]-[Tg ayant ladite structure de chaîne glucidique spécifique]-[protéine-2] et un conjugué (b) de [protéine-(1) marquée]-[Tg ayant une structure de chaîne glucidique autre que ladite structure de chaîne glucidique spécifique],
(ii) séparer chacun des conjugués de la protéine (1) marquée libre, et
(iii) mesurer respectivement les quantités des substances de marquage dans chacun du conjugué (a) et du conjugué (b) formés dans l'étape (i), d'où il résulte l'obtention des quantités de Tg ayant ladite structure de chaîne glucidique spécifique, de Tg ayant une structure de chaîne glucidique autre que la structure de chaîne glucidique spécifique et de Tg totales ;

(8) :

(i) faire réagir l'échantillon avec une protéine-(2), qui est un anticorps ou une lectine capable de se lier spécifiquement à ladite structure de chaîne glucidique spécifique de Tg, et une protéine-(3) marquée, qui est un anticorps anti-Tg ou un récepteur capable de se lier à toutes les Tg indépendamment du fait que la protéine-(2) y soit déjà liée ou non, et une protéine-(4), qui est un anticorps anti-Tg ou un récepteur capable de se lier à Tg mais incapable de se lier à Tg à laquelle la protéine-(2) est déjà liée, afin de former un conjugué (a) de [protéine-(3) marquée]-[Tg ayant ladite structure de chaîne glucidique spécifique]-[protéine-(2)], et un conjugué (b) [protéine-(3) marquée]-[Tg ayant une structure de chaîne glucidique autre que ladite structure de chaîne glucidique spécifique]-[protéine-(4)],
(ii) ôter la protéine-(3) marquée libre et séparer les conjugués (a) et (b), et
(iii) mesurer les quantités des substances de marquage dans chacun du conjugué (a) et du conjugué (b) formés dans l'étape (i), d'où il résulte l'obtention des quantités de Tg ayant ladite structure de chaîne glucidique spécifique, de Tg ayant une structure de chaîne glucidique autre que ladite structure de chaîne glucidique spécifique, et de Tg totales ;

(9) :

(i) faire réagir l'échantillon avec une protéine-(1) marquée, qui est un anticorps anti-Tg capable de se lier à une région constante de Tg ou un récepteur capable de se lier à Tg, et une protéine-(2), qui est un anticorps ou une lectine capable de se lier spécifiquement à ladite structure de chaîne glucidique spécifique de Tg et une protéine-(4), qui est un anticorps anti-Tg ou un récepteur capable de se lier à Tg mais incapable de se lier à Tg à laquelle la protéine-(2) est déjà liée, afin de former un conjugué (a) de [protéine-(1) marquée]-[Tg ayant ladite structure de chaîne glucidique spécifique]-[protéine-(2)], et un conjugué (b) de [protéine-(1) marquée]-[Tg ayant une structure de chaîne glucidique autre que ladite structure de chaîne glucidique spécifique]-[protéine-(4)],
(ii) ôter la protéine-(1) marquée libre et séparer les conjugués (a) et (b), et
(iii) mesurer les quantités des substances de marquage dans chacun des conjugués (a) et (b) formés dans l'étape (i), d'où il résulte l'obtention des quantités de Tg ayant ladite structure de chaîne glucidique spécifique, de Tg ayant une structure de chaîne glucidique autre que ladite structure de chaîne glucidique spécifique et de Tg totales ;

(B) de mesurer la quantité de Tg totales par l'un quelconque des procédés i) à ii) ci-dessous :

(i) un dosage immunologique utilisant une protéine de liaison à Tg, et
(ii) (7) à (9) comme défini dans (A) ;

(C) de calculer le rapport de la quantité de Tg ayant ladite structure de chaîne glucidique spécifique présente dans un échantillon, sur la quantité de Tg totales, ou le rapport de la quantité de Tg ayant une structure de chaîne glucidique autre que ladite structure de chaîne glucidique spécifique, sur la quantité de Tg totales; et
(D) de déterminer la malignité de la tumeur de la thyroïde en comparant le rapport calculé avec un rapport prédéterminé correspondant provenant d'un échantillon liquide de référence originaire d'un corps vivant ayant une thyroïde normale ou une thyroïde bénigne,

dans lequel l'échantillon est déterminé comme étant malin lorsque le rapport calculé est significativement différent de celui de l'échantillon liquide de référence de la thyroïde normale et est significativement différent de celui de

l'échantillon liquide de référence de la thyroïde bénigne, ou lorsque le rapport calculé est significativement différent de celui de l'échantillon liquide de référence de la thyroïde bénigne et n'est pas significativement différent de celui de l'échantillon liquide de référence de la thyroïde normale.

**2.** Procédé selon la revendication 1, dans lequel la lectine est capable de se lier au D-galactose ou à la N-acétyl-D-galactosamine ou de se lier au D-mannose.

**3.** Procédé selon la revendication 1, dans lequel la structure de chaîne glucidique incapable de se lier à la lectine est trouvée dans une thyroglobuline qui est produite par une cellule de carcinome.

**4.** Procédé selon la revendication 3, dans lequel la cellule de carcinome provient d'un carcinome de la thyroïde.

**5.** Utilisation de (a) un anticorps anti-Tg capable de se lier à une région constante de Tg et/ou un récepteur capable de se lier à Tg, et (b) un anticorps et/ou une lectine capables de se lier spécifiquement à une structure de chaîne glucidique spécifique de Tg, dans laquelle la structure de chaîne glucidique spécifique est une structure de chaîne glucidique de Tg capable de se lier à une lectine choisie parmi la concanavaline A, l'agglutinine de *Lens culinaris* et l'agglutinine de *Ricinus communis*, dans un procédé destiné à déterminer une malignité d'une tumeur de la thyroïde comme défini dans la revendication 1.

**6.** Réactif destiné à déterminer une malignité d'une tumeur de la thyroïde qui comprend :

(1) un anticorps anti-Tg capable de se lier à une région constante de Tg et éventuellement un récepteur capable de se lier à Tg, et
(2) une lectine ou un anticorps capable de se lier à une structure de chaîne glucidique spécifique, dans laquelle la structure de chaîne glucidique spécifique est une structure de chaîne glucidique de Tg capable de se lier à une lectine choisie parmi la concanavaline A, l'agglutinine de *Lens culinaris* et l'agglutinine de *Ricinus communis*.

Fig. 1

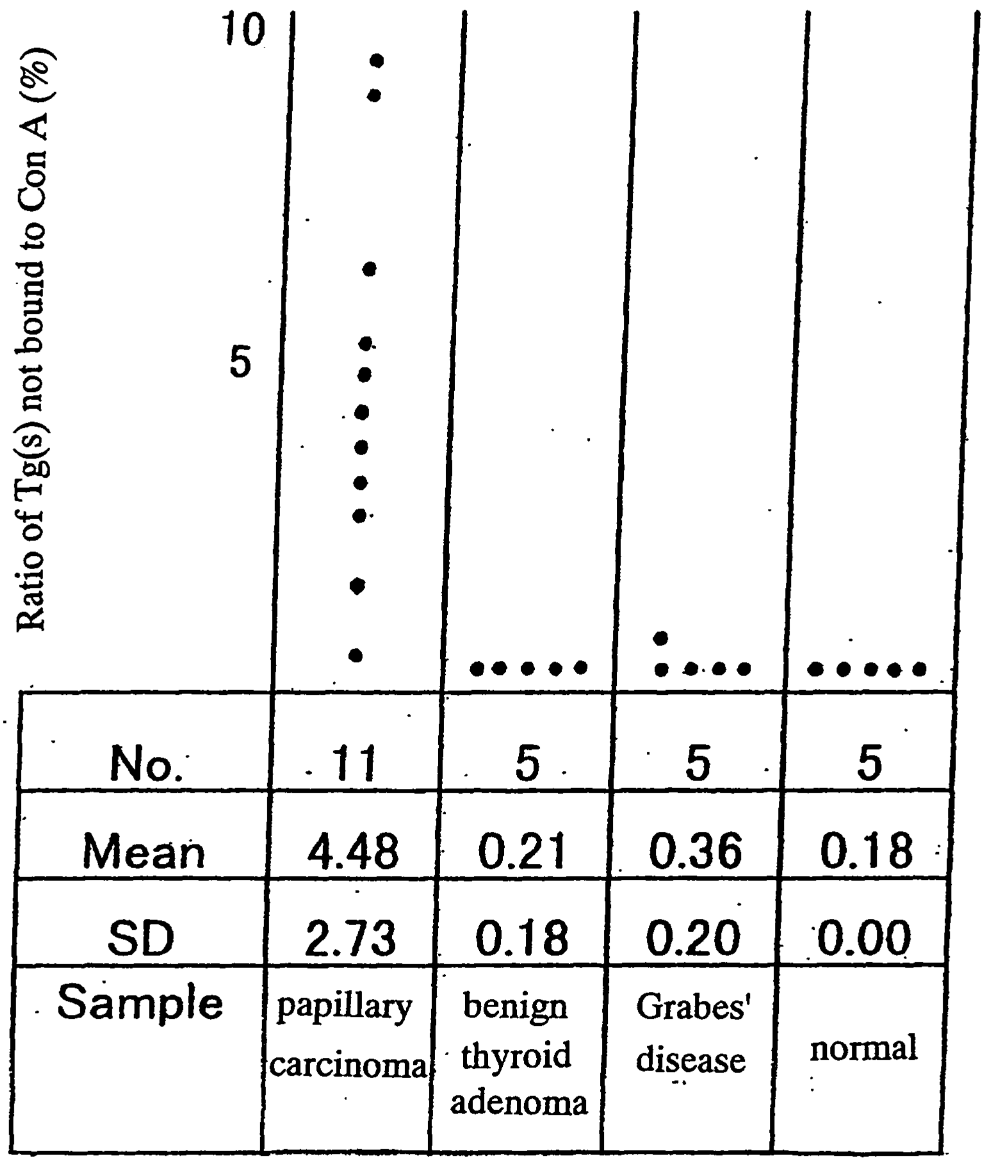


| | | | | |
|---|---|---|---|---|
| No. | 11 | 5 | 5 | 5 |
| Mean | 4.48 | 0.21 | 0.36 | 0.18 |
| SD | 2.73 | 0.18 | 0.20 | 0.00 |
| Sample | papillary carcinoma | benign thyroid adenoma | Grabes' disease | normal |

Fig. 2

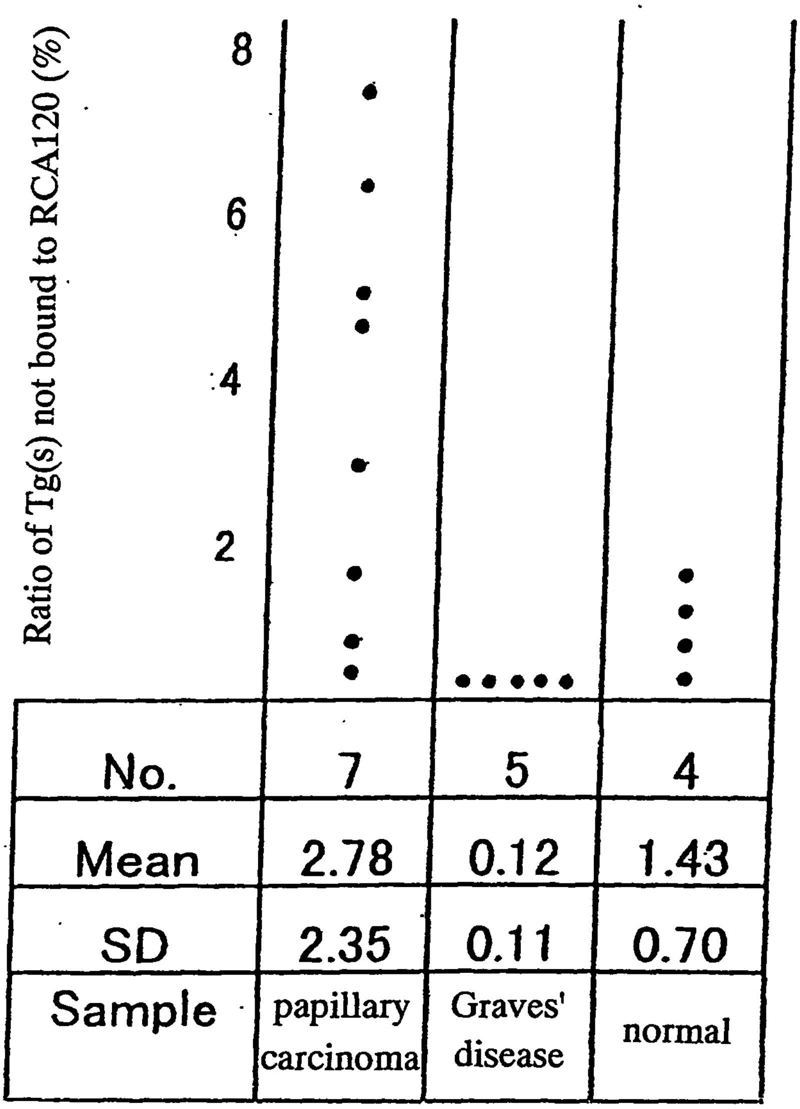


| No. | 7 | 5 | 4 |
|---|---|---|---|
| Mean | 2.78 | 0.12 | 1.43 |
| SD | 2.35 | 0.11 | 0.70 |
| Sample | papillary carcinoma | Graves' disease | normal |

Fig. 3

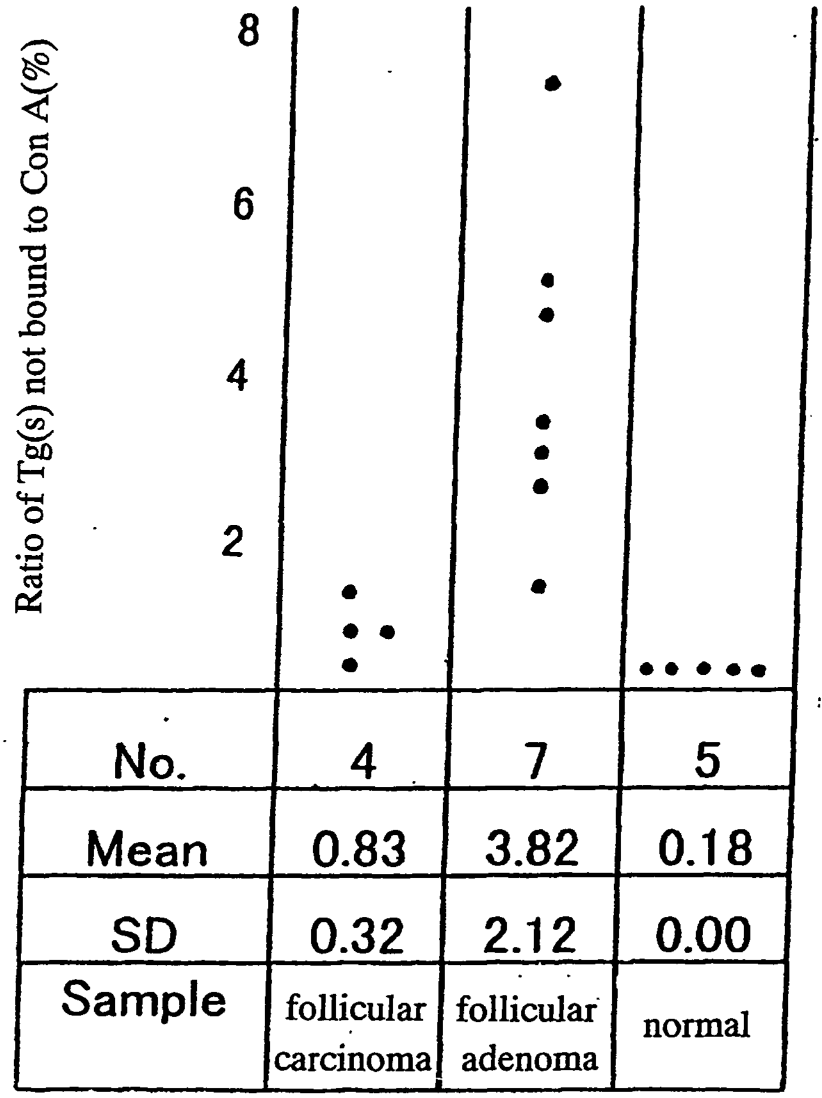


| No. | 4 | 7 | 5 |
|---|---|---|---|
| Mean | 0.83 | 3.82 | 0.18 |
| SD | 0.32 | 2.12 | 0.00 |
| Sample | follicular carcinoma | follicular adenoma | normal |

Fig. 4

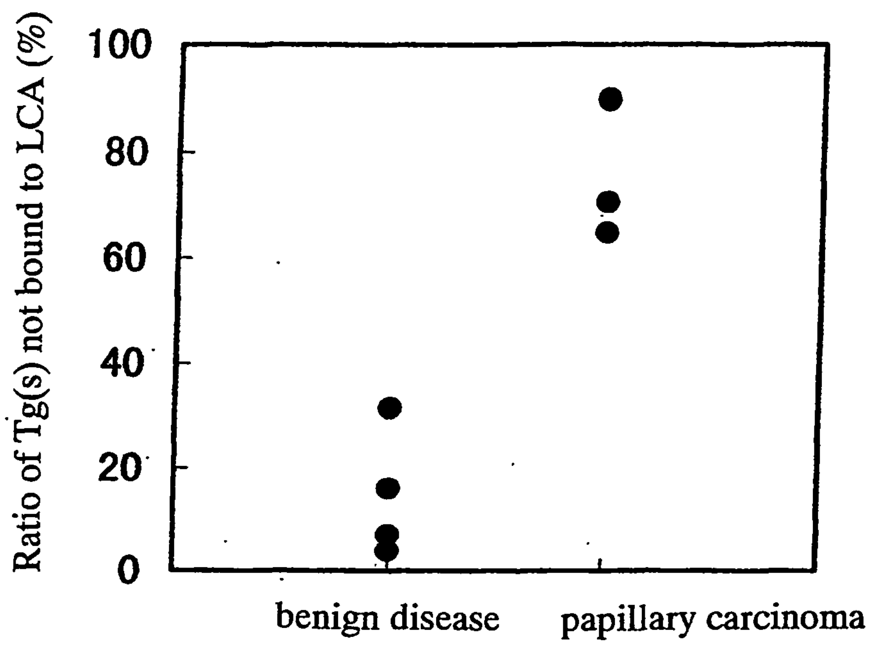
Ratio of Tg(s) not bound to LCA (%)
100
80
60
40
20
0
benign disease
papillary carcinoma

Fig. 5

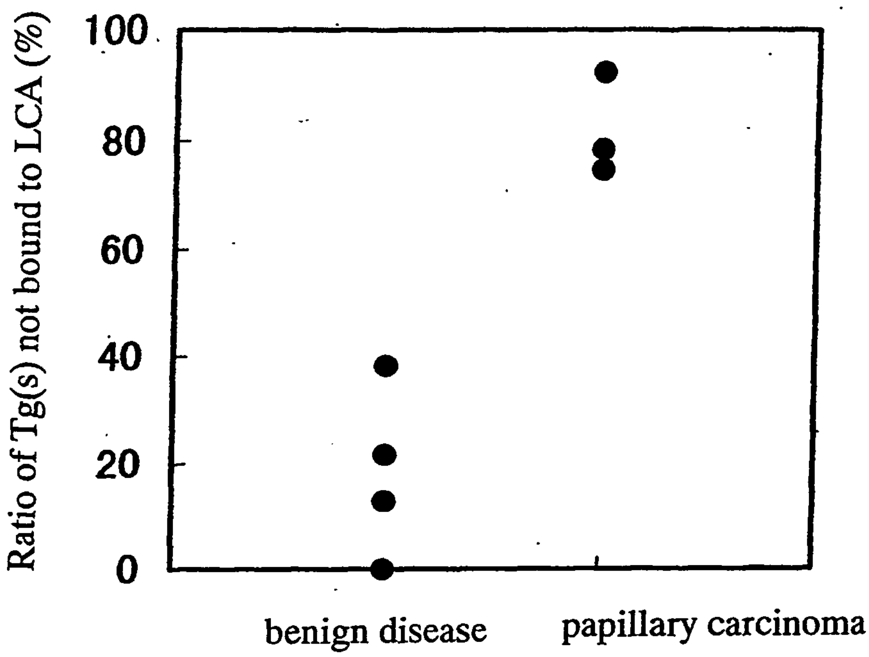
Ratio of Tg(s) not bound to LCA (%)
100
80
60
40
20
0
benign disease
papillary carcinoma

Fig. 6

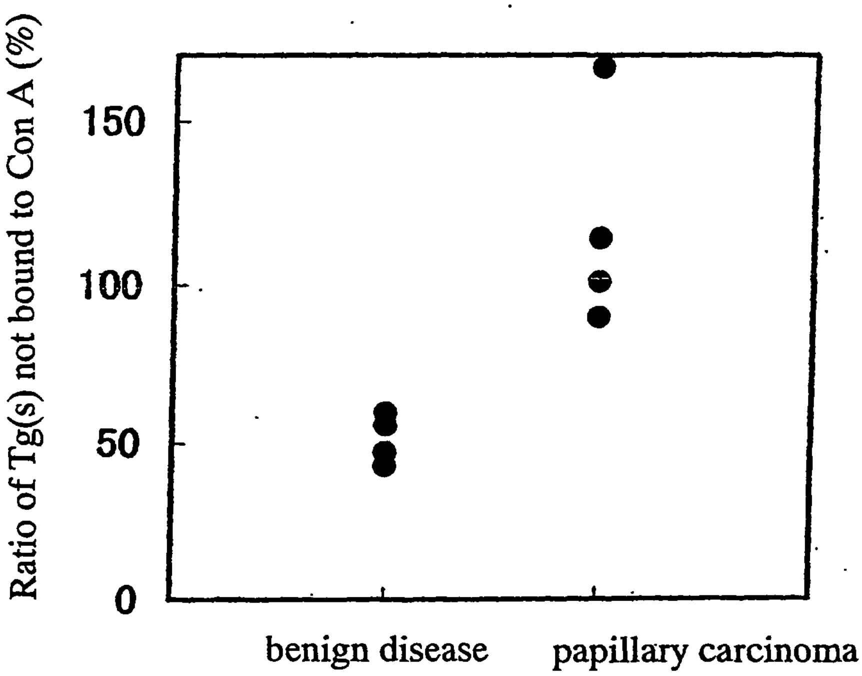
Ratio of Tg(s) not bound to Con A (%)
150
100
50
0
benign disease
papillary carcinoma